# EUROPEAN PATENT SPECIFICATION

(11) **EP 2 288 618 B1**
(45) Date of publication and mention of the grant of the patent: **20.09.2017**
(21) Application number: 08749431.6
(22) Date of filing: 09.05.2008
(51) Int. Cl.: C07K 14/08, C12N 7/04

(54) **CHIMERIC FUSION PROTEINS AND VIRUS LIKE PARTICLES FROM BIRNAVIRUS VP2**
CHIMÄRE FUSIONSPROTEINE UND VIRUS-ÄHNLICHE PARTIKEL AUS BIRNAVIRUS VP2
PROTÉINES DE FUSION CHIMÈRES ET PARTICULES DE TYPE VIRAL DE BIRNAVIRUS VP2

(43) Date of publication of application: 02.03.2011
(73) Proprietor: Chimera Pharma S.l.u., 28760 Tres Cantos (ES)
(72) Inventor: ZÜRCHER, Thomas, 28760 Tres Cantos (ES); BERNAL, Juan José, 28760 Tres Cantos (ES); VON KOBBE, Cayetano, 28760 Tres Cantos (ES); JIMÉNEZ TORRES, Ignacio, 28760 Tres Cantos (ES); DIAZ BLÁZQUEZ, Ana, 28760 Tres Cantos (ES); MARTIN LORENZO, Diana, 28760 Tres Cantos (ES); CALDERITA LUCAS, Gloria, 28760 Tres Cantos (ES); GARCIA DE CASTRO, Arcadio, 28760 Tres Cantos (ES)
(74) Representative: Pons
(86) International application number: PCT/EP2008/003778
(87) International publication number: WO 2009/135518

(56) References cited:
- WO-A-02/088339
- WO-A-2005/071069
- WO-A-2007/009673
- US-A- 5 788 970
- VAN LOON A A W M ET AL: "Alteration of amino acids in VP2 of very virulent infectious bursal disease virus results in tissue culture adaptation and attenuation in chickens" JOURNAL OF GENERAL VIROLOGY, vol. 83, no. 1, January 2002 (2002-01), pages 121-129, XP002514415 ISSN: 0022-1317
- CHEVALIER CHRISTOPHE ET AL: "Structural peptides of a nonenveloped virus are involved in assembly and membrane translocation" JOURNAL OF VIROLOGY, vol. 79, no. 19, October 2005 (2005-10), pages 12253-12263, XP002514416 ISSN: 0022-538X
- LETZEL TOBIAS ET AL: "Molecular and structural bases for the antigenicity of VP2 of infectious bursal disease virus" JOURNAL OF VIROLOGY, vol. 81, no. 23, December 2007 (2007-12), pages 12827-12835, XP002514417 ISSN: 0022-538X

## Description

### TECHNICAL FIELD

The field of the invention refers to chimeric Virus Like Particles (VLP) derived from the Birnavirus chimeric VP2 protein. In particular, the present disclosure refers to chimeric VP2 fusion proteins which incorporate insertions and/or substitutions with one or more particular peptides of interest while maintaining the capacity to assemble in the form of VLP. The invention identifies particular insertion and/or substitutions sites within VP2 P loop regions and outside said P loop regions. The invention also incorporates methods for the identification of preferred insertion and substitution sites within VP2 for the incorporation of particular peptides of interest. The resulting chimeric VLP are of interest in the design of therapeutic and prophylactic vaccines as well as in the design of drug delivery systems, carriers for DNA and RNA in gene therapy, as targeted agents, in the development of antitoxins, and as diagnostic reagents.

### BACKGROUND OF THE INVENTION

Virus Like Particles (VLP) are nanometric structures resulting from the assembly of structural viral proteins. These particles resemble the virus from which they were derived but lack viral nucleic acid and are therefore not infectious. Virus Like Particles (VLP) are preferred forms in the design of vaccines and in other applications in human health and diagnostics.

Vaccines most often incorporate VLP that are derived from the causative agents of the disease as is exemplified by Hepatitis B VLP useful in vaccination against Hepatitis. However, VLP may be made to incorporate unrelated/heterologous peptides relevant to disease. These chimeric VLP help in antigen presentation and in promoting an immune response in the receiving subject. An example being VLP formed by hepatitis B core and surface antigen fused to the Malaria or HCV epitope, respectively [Grgacic E. et al. (2006) Methods 40(1):60-65]. Maintenance of VLP structure is an essential feature in the design of these agents.

VLP three dimensional nanometric structures not only provide the means for incorporating antigens for their improved presentation to the immune system but are also useful in the design of drug delivery systems [Georgens C. et al. (2005) Current Pharmaceutical Biotech. 6(1):49-55], as carriers for DNA in gene therapy [Ou WC. et al. (2001) J. Med. Virol., 64(3):366-373; and Krauzewicz N. et al (2000) Gene Therapy 7(13):1094-1102], as targeted agents [Gleiter S. and Lilie H. (2001) Protein Science 10(2):434-444], in the development of antitoxins [Manayani DJ. et al. (2007) PLoS Pathogens 3(10):1422-1431] and as diagnostic reagents [Martinez-Torrecuadrada JL. et al. (2000) Clinical Diagnostic Lab. Immunol. 7(4):645-651]. Again, maintenance of VLP structure is a common and essential feature in the design of these agents.

Commonly described VLP include those derived from Hepatitis B, Papilloma, Polyoma and other viruses. Other VLP described include those derived from Infectious Bursal Disease Virus (IBDV).

IBDV belongs to the *Birnaviridae* family and is the causative agent of Gumboro disease in poultry. Wild-type IBDV particles are icosahedral, with T=13 symmetry and a single protein shell formed by 260 trimers of the VP2 protein (37 kDa). The inner side of the VP2 shell appears to be supported by a scaffold formed by 200 trimers of the VP3 protein (29 kDa). It has been suggested that a third protein, VP4 (28 kDa), may also play a scaffolding role. In normal virus assembly, protein components result from the proteolytic processing of a larger polypeptide pVP2-VP4-VP3 precursor (109 kDa). This precursor undergoes auto-catalysis to release a 512 amino acid VP2 precursor (pVP2), VP4 and VP3 polypeptides. VP4 belongs to the *Lon* protease family and is responsible for the proteolytic cleavage while pVP2 and VP3 polypeptides are directly responsible for capsid assembly. A final cleavage of pVP2 at its C-terminal end gives rise to the mature 441 amino acid form of VP2 found in the virion [Da Costa B. et al. (2002) J. Virology 76(5):2393-2402]. VP2 proteins found in different IBDV strains have been reported to present a protein sequence homology of over 80%. VP2 proteins of other Birnaviridae share homologies with IBDV of 40% for aquatic Birnavirus and 30 % for Drosophila Birnavirus [Coulibaly F. et al. (2005) Cell 25,120(6):761-772].

It has been found that expression in eukaryotic cells of the IBDV pVP2-VP4-VP3 polyprotein gives rise to the formation of icosahedral T=13 VLP that appear morphologically and biochemically indistinguishable from IBDV capsids and that this process does not require the presence of the viral genome or other proteins encoded by the viral genome, such as VP5 and VP1 [Martinez-Torrecuadrada JL. et al. (2001) J. Virology 75(22):10815-10828].

The ability of IBDV proteins to generate T=13 provides a versatile system for the incorporation of foreign peptides of interest relevant to human disease in the form of a vaccine. This is exemplified by Delmas B. et al. in WO02088339 in which Green Fluorescent Protein (GFP) is engineered as a C-terminal fusion to the precursor polyprotein pVP2-VP4-VP3-GFP to produce T=13 VLP in which GFP is fused to VP3 and presumably located inside the VLP. Similarly, Rodriguez Aguirre JF. et al. in WO2005071069 describe pVP2-VP3-X fusion proteins were a peptide of interest in vaccination (X) is fused to the C-terminus of VP3. Most likely constructs incorporating a peptide of interest fused to VP3 result in icosahedral VLP were the peptide of interest is sequestered within the T=13 particle.

More so, expression of VP2 in insect cells, in the absence of other IDBV proteins, has been found to result in the formation of smaller size iscosahedral T=1 VLP [Martinez-Torrecuadrada JL. et al. (2003) Vaccine 21(17-18):1952-1960]. It has been reported that the expression of VP2 fragments between 441 and 466 amino acids leads to the formation of icosahedral VLP, whereas the longer VP2 fragments between 466 and 501 amino acids tend to form tubular particles [Ruiz Caston J. et al. WO2005105834; and Saugar I. et al. (2005) Structure 13(7):1007-1117]. This has been exploited by Rodriguez Aguirre JF. et al. in WO2007009673 which describe the incorporation of peptides of interest (X) in T=1 VLP produced as VP2-X terminal fusion proteins. Recent reports however suggest that C-terminus fused peptides are not exposed on the VLP surface [Coulibaly F. et al. (2005) Cell 25,120(6):761-772; Lee CC. et al. 2006 J. Struct Biol. 155(1):74-86; and Garriga D. et al. (2006) J. Virol. 80(14):6895-6905] and that purification procedures carried out on C-terminal fusions of VP2 with Histidine residues with a metal ion affinity column are most likely mediated by naturally occurring Histidine residues within VP2 [Doong et al., (2007) Anal. Chem. 79(20):7654-7656]. Therefore, VP2 terminal fusions most likely result in the incorporation of peptides of interest in a sequestered form inside the T=1 VLP. Furthermore high sequence variability is found in the loops of the P domains named BC (AA **219-224**), DE (AA **249-254**), FG (AA **283-287**) and HI (AA **315-324**) which also appear to be the targets of neutralizing antibodies and harbour mutations in escape mutants indicating that these regions are immunogenic [Lee CC. et al. (2006) J. Struct Biology 155(1):74-86].

Therefore, to date, incorporation of peptides of interest in IBDV derived VLP, T=1 and T=13, has focused on VP2 and VP3 terminal fusions that most likely result in sequestration of the peptide of interest inside the VLP, as referred in Rodriguez-Aguirre JF. et al. WO2005071069, and suboptimal presentation to cells, cell surface receptors, soluble factors or diagnostic reagents.

Incorporation by means of insertion or substitution of the peptide of interest within VP2 represents improved alternatives to terminal fusions. In particular, improvements may result from surface exposure of the inserted sequences or the total or partial sequestration within the VLP structure of the inserted peptides. It is recognized that while surface exposure may be necessary for targeting against other biological entities such as cell surface receptors or soluble factors, total o partial sequestration may be desirable to avoid biological degradation or proteolysis or in eliciting a cellular immune response. Therefore, chimeric VLP in which peptides of interest are incorporated in accordance to their intended biological activity could represent improved candidate vaccines, DNA or RNA carriers, targeted agents, diagnostic, imaging, or therapeutic reagents. However, the design of VLP based on IBDV VP2 insertions or substitutions is restricted by the fact that VP2 is a main structural protein of IBDV viral capsid, and insertions or substitutions with particular foreign peptide sequences may result in the inability of the resulting chimeric VP2 protein to self assemble in the form of VLP. In fact, this is clearly exemplified by studies carried out on alternative polyoma VLP [Shin YC. and Folk WR. (2003) J. of Virology 77(21):11491-11498] were the insertion of peptides often result in VLP disruption.

Therefore, the present disclosure relates to chimeric fusion proteins of Birnavirus VP2, or fragments thereof that incorporate one or more insertions, or partial substitutions, with particular peptides of interest, and which are capable of assembling into VLP structures. More so, the present disclosure relates to methods for the identification and selection of preferred insertion sites within VP2 for the incorporation of peptides of interest without loss of VLP structure and with efficient VLP formation.

### DETAILED DESCRIPTION OF THE INVENTION

Virus Like Particles (VLP) are of interest in the design of medicines, therapeutic and prophylactic vaccines as well as in the design of drug delivery systems, carriers for nucleic acids in gene therapy, as targeted agents, imaging agents, in the development of antitoxins and as diagnostic reagents applicable to human and veterinary health. The present disclosure relates to chimeric VLP of chimeric VP2 fusion proteins, incorporating insertions and/or substitutions with one or more peptides of interest and methods for the identification and selection of said chimeric VLP.

"**Peptides of interest**" are hereby defined as amino acid sequences other than IBDV sequences, including vaccine components, antigens and epitopes, targeting sequences, binding sequences, catalytic domains, pharmacology modulators, immunostimulators, toxins and antitoxins which are relevant to human or veterinary health.

"**DNA of interest**" refers to DNA sequences encoding for peptides of interest.

"**VP2**" refers to Infectious Bursal Disease Virus (IBDV) VP2 sequences and proteins, including the 512 amino acid VP2 precursor protein (pVP2), the mature 441 VP2 protein, or a fragment of at least 400 amino acids thereof, capable of forming VLP. VP2 includes any VP2 protein found in different IBDV strains, with special reference to those with a protein sequence homology of at least 80% between them. VP2 protein also refers to other Birnaviridae VP2 proteins with protein sequence homologies over 30%, preferably over 40%, and more preferably over 60% with those of IBDV.

"**P loop regions**" refer to the four loops of the IBDV VP2 P domain named BC (Q₂₁₉-G₂₂₄), DE (R₂₄₉-G₂₅₄), FG (T₂₈₃-D₂₈₇) and HI (S₃₁₅-Q₃₂₄). In brackets the first and last amino acids and corresponding position within VP2 sequence. All other locations within VP2 excluding the C- and N-terminal amino acid are referred to as "**Outside P loop regions**".

"**Chimeric VP2 fusion proteins**" refer to chimeric VP2 proteins incorporating one or more insertions and/or substitutions, at locations other than the C- and N-terminus, with one or more peptides of interest other than IBDV sequences.

"**DNA vectors**" refer to DNA sequences that facilitate cloning and expression of VP2 incorporating DNA of interest at the desired insertion or substitution sites. DNA constructs also incorporate "**DNA expression vectors**" that when expressed in an appropriate host such as bacteria, yeast, insect cells, plants, or mammalian cells, result in VP2, VP2 fusion proteins incorporating peptides of interest, and other IBDV proteins. In the description of said DNA vectors, the insert or substitution is defined in brackets () with arrows ↑ representing the incorporation point of particular peptide of interest X and flanking amino acid positions within the VP2 sequence [e.g.: pESC-URANP2(Q₂₁₉ ↑X↑Y₂₂₀)]. Deletions are represented with a triangle followed by flanking elements of the deleted VP2 peptide sequence [e.g.: pESC-URA/VP2/Δ Y₂₂₀ - G ₂₂₃]. In the definition of multiple lysine (K) substitutions each of the substituted amino acid positions is followed by a K and separated by a hyphen - [e.g.: pESC-URA/VP2(Q₂₂₁K - H ₂₅₃K-G₂₈₅K)].

"**Chimeric VP2 VLP**" refer to T=1 and T=13 VLP and other nanostructures resulting from the assembly of chimeric VP2 fusion proteins and optionally incorporating IBDV VP3 proteins or fusion proteins thereof.

"**VP2-VLP antibodies**" refer to anti-VP2 antibodies that are specific for VP2 and VP2 fusion proteins assembled as VLP.

"**VLP formation**" is determined upon expression of DNA constructs coding for VP2 fusion proteins in the appropriate expression system and quantification by means of a VP2-VLP enzyme linked immunoassay **"VLP-ELISA"** which makes use of anti-VP2 antibodies capable of recognising VP2 only when assembled as VLP. "**VLP formation efficiency**" is calculated as a percentage of VLP formation in comparison with that of the native 452 amino acid VP2. Generally, VLP-ELISA values below 20% are regarded as background (BG), and associated chimeric VP2 fusion proteins are considered as not resulting in efficient VLP formation. While VLP formation efficiency values of 20% or higher are regarded as being compatible with VLP formation, that is sufficient for efficient VLP formation, and insertion sites of associated chimeric VP2 fusion proteins can be considered as preferred insertion sites, VLP formation efficiencies above 50% and preferably above 70% are recognised as a desirable feature.

"**Preferred insertion and/or substitution sites**" refer to locations within VP2 at which the incorporation of peptides of interest result in higher VLP formation efficiencies.

IBDV VP2 protein is naturally folded into a helical base (B) domain, a shell (S) domain, and a projection (P) domain and can be made to spontaneously assemble into trimer subunits to form icosahedral VLP T=1, or T=13 in the presence of VP3. In the present invention it has been observed that the insertion of a peptide of interest within IBDV VP2 often results in destabilisation of VLP structure. Furthermore it has been observed that for many possible insertion or substitution sites, VLP formation efficiency of the resulting chimeric VP2 fusion proteins depends on the sequence of the particular peptide of interest inserted. The present disclosure is directed towards the identification of preferred sites within VP2 which are appropriate for the insertion or substitution with peptides of interest while maintaining VLP structure, methods for the identification of said preferred insertion or substitution sites, and the resulting chimeric VP2 VLP.

Furthermore, the present disclosure is directed towards chimeric VP2 VLP incorporating one or more insertions or substitutions with peptides of interest within the four VP2 P loops, BC, DE, FG and HI and/or locations outside said P loops.

Most of the particular embodiments of the present invention, have been exemplified (examples 1 to 15) with five different example peptides selected for the purpose of this invention, namely TS (SEQ. ID. NO: 1), Flag (SEQ ID NO 2), cMyc (Seq SEQ ID NO 3), V5 (SEQ ID NO 4) and VSV-G (SEQ ID NO 5).

### Chimeric VP2 fusion proteins incorporating insertions and/or substitutions within P loop regions.

The BC, DE, FG and HI loops of the IBDV VP2 P domain represent possible insertion sites for fusion proteins incorporating peptides of interest. This is explored in the present invention through the incorporation of five different example peptides of interest, namely TS (SEQ. ID. NO: 1), Flag (SEQ. ID. NO: 2), cMyc (SEQ. ID. NO: 3), V5 (SEQ. ID. NO: 4) and VSV-G (SEQ. ID. NO: 5), at all the possible insertion points within VP2 P loops as shown in Example 1.

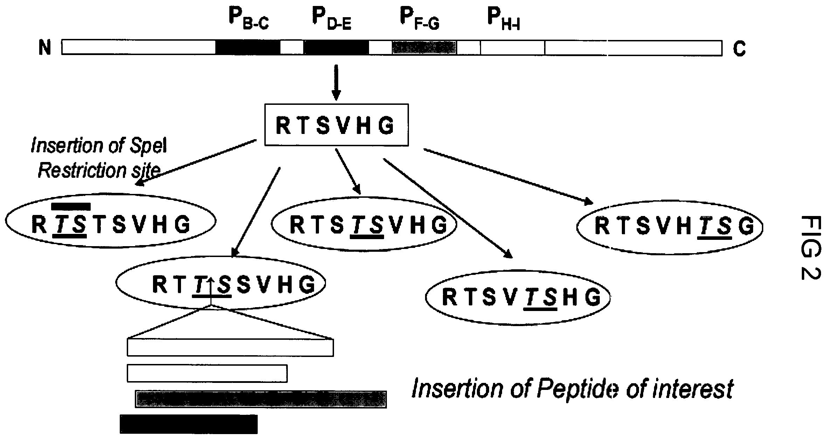

Incorporation of the Threonine-Serine (SEQ. ID. NO: 1) sequence coding for a *Spe*I restriction site at all possible insertion sites within the BC, DE, FG and HI P loop regions and closely adjacent positions resulted in the identification of preferred insertion points. Many insertion points within the P loop regions appeared to be compatible with VLP formation. As depicted in Figure 2, incorporation of the *Spe*I restriction site within the TS sequence facilitates subsequent incorporation of DNA sequences encoding for the other example peptides of interest, namely Flag (SEQ. ID. NO: 2), cMyc (SEQ. ID. NO: 3), V5 (SEQ. ID. NO: 4) and VSV-G (SEQ. ID. NO: 5). Insertion of these different model peptides of interest, at each of the possible VP2 P loop region positions, identified suitable insertion sites and demonstrated that preferred sites within the BC, DE, FG and HI P loops varied according to the inserted DNA of interest. Generally the incorporation of peptides of interest in VP2 regions adjacent to the BC, DE, FG and HI P loops does not result in significant VLP formation efficiency and thus does not appear to be compatible with VLP formation.

Furthermore, as demonstrated in Example 2, VP2 of different lengths, exemplified by IBDV VP2 of different lengths at their C-terminal, namely VP2 with 452, 441 and 456 amino acids (VP2 452, VP2 441 and VP2 456), also permit the insertion of the example peptides of interest, namely Flag (SEQ. ID. NO: 2) and cMyc (SEQ. ID. NO: 3), into P loop regions with little variation of VLP formation efficiency. Generally insertion into VP2 456 resulted in a reduction of VLP formation efficiency compared to VP2 452 or VP2 441 with the same insertion. VP2 452 and VP2 441 both represent preferred lengths for the formation of VLP incorporating peptides of interest. It is envisaged that VP2 proteins of at least 400 amino acids, other than 452, 441 and 456, may also be capable of effectively forming VLP and chimeric VP2 VLP.

Therefore, the present disclosure incorporates chimeric VP2 fusion proteins and resulting chimeric VP2 VLP where peptides of interest are inserted at the BC (Q₂₁₉-G₂₂₄), DE (R₂₄₉-G₂₅₄), FG (T₂₈₃-D₂₈₇) and HI (S₃₁₅-Q₃₂₄) P loop regions of VP2 protein and fragments thereof. The present disclosure also incorporates the DNA vectors and constructs that permit cloning DNA of interest at each of the available sites within the VP2 P loop regions and DNA expression vectors for the expression of the resulting chimeric VP2 P loop region fusion proteins.

IBDV VP2 P loop regions not only represent potential insertion sites for peptides of interest but also represent possible sites for the substitution of VP2 amino acids for peptides of interest. Locations and/or structural elements of VP2 which have been shown to be compatible with an insertion may also be considered as potential sites for substitutions with peptides of interest. Substitution of one or more amino acid residues adjacent, or close, to the insertion site may be explored or alternatively entire structural elements, such as a connecting loop, or parts of it may be substituted. VP2 P loop region substitutions are exemplified in Example 3 where amino acids within the BC, DE, FG and HI VP2 P loop regions are substituted by example peptides of interest, namely Flag (SEQ. ID. NO: 2) and cMyc (SEQ. ID. NO: 3), and resulting constructs evaluated for their capacity to form VP2 VLP. Removal of the P loop regions while maintaining the first and last residue of the P loop regions appeared to be compatible with VLP formation. Furthermore, although with a lesser, but still equal or above 20% efficiency, for two of the expression vectors and inserted DNA of interest, the substitution of an entire P loop region also resulted in VLP formation depending on the site of substitution. Therefore, incorporation of peptides of interest within VP2 can be directed towards the substitution of the entire P loop regions, or preferably only parts of them. Furthermore, the peptides of interest introduced as substitutions in the BC, DE, FG and HI VP2 P loop regions may be of same or different length than the P loop regions for which they have been substituted.

The present disclosure therefore incorporates chimeric VP2 fusion proteins and chimeric VP2 VLP resulting from the substitution of VP2 P loop regions, or fragments thereof, by particular peptides of interest at one or more locations within the P loop regions. The present disclosure also incorporates the DNA vectors and constructs that permit cloning DNA of interest at each of the available substitution positions within the VP2 P loop regions, and DNA expression vectors for the expression of the resulting chimeric VP2 P loop fusion proteins.

An example of reference of VLP resulting from VP2 fusion proteins is Example 4 which describes the substitution with lysine (K) residues at different points within BC, DE, FG and HI VP2 P loop regions and the chemical conjugation of the example peptide cMyc (SEQ. ID. NO: 3). Incorporation of K residues in VP2 P loop regions permits the chemical conjugation of the resulting VLP with multiple copies of biological and chemical entities containing, or made to contain, cysteine residues at the desired conjugation points. The incorporation of K residues may involve the insertion or substitution of VP2 amino acid residues by K, poly K, or K rich peptides, at the VP2 P loop regions or outside said P loops. Furthermore, desired conjugation points within VP2 may be substituted, or made to contain, amino acid sequences, other than K residues, that facilitate chemical conjugation with biological and chemical entities by other means of conjugation or coupling such as, but not limited to, cysteines, tyrosine, histidine, glutamic acid, or aspartic acid. Preferred substitution and/or insertion sites for K residues or other residues that permit conjugation include those found to favour higher VLP formation efficiency.

The present disclosure therefore incorporates chimeric VP2 VLP resulting from the insertion or substitution of VP2 amino acid residues by residues that facilitate chemical conjugation of biological and chemical entities that may contribute towards the desired biological or pharmacological properties of the chimeric VLP. The present disclosure also incorporates DNA vectors and DNA expression vectors for the expression of chimeric VP2 VLP incorporating amino acid residues that facilitate chemical conjugation.

### Chimeric VP2 fusion proteins incorporating insertions and/or substitutions outside P loop regions.

Potential insertion of foreign peptides in the B, S or P domains of VP2 outside the BC (AA **219-224**), DE (AA **249-254**), FG (AA **283-287**) and HI (AA **315-324**) P loop regions, most likely result in disruption of VLP structure but may also represent a means for modulating surface exposure of the inserted peptides of interest or their biological activity. In the present disclosure regions outside the VP2 P loop regions which permit the incorporation of particular peptides of interest by insertion or substitution without loss of VLP structure are identified by means of transponson insertion mutagenesis using transposons Tn5 and Mu.

As shown in Example 5, a random screen with Tn5 generated a DNA library of clones with Tn5 insertions along the complete VP2 452 amino acid sequence. Evaluation of the capacity of these constructs to produce VLP resulted in the identification of a number of insertion sites that result in VP2 insertion fusion proteins that retain VLP structure. Similarly, as shown in Example 6, a random screen with transposon Mu generated a DNA library of clones with insertions along the complete VP2 452 amino acid sequence. Evaluation of the capacity of these constructs to produce viable VLP lead to the identification of a number of potential insertion sites that result in VP2 insertion fusion proteins that retain VLP structure.

As exemplified in Examples 5 and 6, insertion locations identified with Tn5 and Mu transposon mutagenesis vary depending on the transposon sequences used. Other transposons or means for random insertion mutagenesis may also be used for the identification of additional sites within VP2 with potential for the insertion and/or substitution with peptides of interest. Transposon insertion mutagenesis permits the identification of locations within the VP2, or chimeric VP2 fusion proteins, which can accommodate insertions and or substitutions while maintaining VLP forming capacity. It is envisaged that by exhaustive evaluation of random insertion libraries, and by means of using different transposons, all possible VP2 insertion sites compatible with VLP formation can be identified. As previously described, those identified VP2 insertion sites may also be considered as potential sites for substitutions with peptides of interest. Identified transposon insertion sites represent potential insertion sites for DNA of interest. As shown in Example 7, insertion of said DNA of interest may be carried out into the inserted transposon sequences, or through the substitution of the inserted transposon sequences. In said Example 7, the presence of a restriction enzyme site such as *Not*I within the inserted Tn5 and Mu transposons facilitates the insertion of example DNA of interest, namely Flag (SEQ. ID. NO: 2) and cMyc (SEQ. ID. NO: 3), into the inserted transposon sequences. However, this results in inserts which may contain undesirable sequences derived from the originally inserted transposon. Alternatively, the identification of the transposon insertion site permits the introduction of a unique cloning site, such as a *Spe*I, at the identified location in VP2. As shown in Example 7, the unique cloning site permits the insertion of DNA of interest and generally results in increased VLP formation efficiencies compared to chimeric VP2 fusion proteins which conserve transposon derived sequences. Therefore in a preferred embodiment of the present disclosure a unique cloning site is engineered at locations within VP2, or chimeric VP2 fusion proteins, originally identified by transposon mutagenesis or other means of random insertion of DNA of interest. Resulting VP2 insertion vectors can then be used for the incorporation of DNA of interest at the desired insertion point for their expression in appropriate expression systems and evaluation of VLP forming capacity. If desired, chimeric VP2 fusion proteins showing the best formation efficiencies or desired properties can be further optimised through the removal of the TS sequences resulting from the engineered unique cloning site by standard genetic engineering techniques. It is acknowledged that the incorporation of DNA of interest can also result in the total or partial substitution of VP2 amino acids closely adjacent to the identified location site.

Therefore, the present disclosure incorporates chimeric VP2 VLP resulting from the assembly of chimeric VP2 fusion proteins which incorporate insertions and/or substitutions with peptides of interest at locations outside the VP2 P loop regions. Furthermore, the present disclosure also incorporates VP2 DNA vectors and DNA expression vectors incorporating one or more insertions and/or substitutions with DNA of interest at locations outside the P loop regions.

### Chimeric VP2 fusion proteins incorporating multiple insertions and/or substitutions.

The identification of preferred insertion and/or substitution sites provides the means for insertion or substitution with peptides of interest at more than one site within VP2. This is exemplified in Example 4 were Lysine (K) residues have been inserted at 1, 2, 3 or 4 locations simultaneously while maintaining the overall VLP structure. Substitution with multiple Lysine residues facilitates the conjugation with multiple copies of a biological or chemical entity, such as nucleic acids, peptides, carbohydrates and small molecules, which may be a desirable feature for purification, targeting, drug loading or in altering VLP surface chemistry for improved pharmacology. Another example of chimeric VP2 VLP resulting from the incorporation of peptides of interest at more than one point within VP2 is provided by Example 8 in which example peptides of interest, cMyc (SEQ. ID. NO: 3) and Flag (SEQ. ID. NO: 2), are inserted and/or substituted at more than one location within VP2. Introduction of peptides of interest can be carried out by the introduction of cloning sites at the desired insertion and/or substitution points, by cloning insertion containing fragments, or following other standard molecular biology procedures. Therefore the present disclosure incorporates chimeric VP2 fusion proteins, and resulting chimeric VP2 VLP, incorporating more than one insertion and/or substitution and DNA expression vectors for expression of said chimeric VP2 fusion proteins.

### Chimeric VP2 fusion proteins incorporating insertions and/or substitutions and terminal fusions.

Furthermore, another object of the present disclosure incorporates VLP in which chimeric VP2 insertion or substitution fusion proteins are in addition also fused, either at their carboxy (C-) or amino (N-) terminal end, to a peptide of interest which may be the same or different to the inserted peptides of interest. Chimeric VP2 insertion or substitution fusion proteins with additional terminal fusions are exemplified in Example 9 were example peptides of interest, Flag (SEQ. ID. NO: 2) and cMyc (SEQ. ID. NO: 3), are inserted at various points within VP2 P loop regions and in addition also fused at the VP2 C- or N- terminus. VLP formation efficiency was found to depend on both, the location of the primary insertion, and the inserted peptide. Furthermore, incorporation of the additional terminal fusion generally results in decreased, but still equal or above 20%, VLP formation efficiencies compared to single insertion chimeric VP2 fusion proteins. As shown in Example 9, for the particular peptides of interest evaluated, some constructs incorporating both, the insertion and terminal fusion, appeared to be compatible with VLP formation.

The present disclosure therefore incorporates chimeric VP2 VLP resulting from VP2 insertion or substitution fusions with peptides of interest and additionally, the terminal fusion of the resulting insertion or substitution with same or different peptides of interest. Identification of the preferred DNA constructs incorporating insertions and terminal fusions may be carried out following the initial identification of the preferred insertion points followed by a C- or N- terminal fusion, or alternatively the terminally fused chimeric VP2 protein may be screened or evaluated for preferred insertion or substitution points. This screen may be carried out against pre-selected sites within VP2, or randomly following transposon mutagenesis or other random cloning approaches.

The present disclosure also incorporates the DNA vectors and constructs that permit cloning DNA of interest at each of the available insertion or substitution positions within a VP2 protein that is also terminally fused, either at their carboxy (C-) or amino (N-) terminal end, to a peptide of interest which may be the same, or different, to the inserted or fused peptides of interest.

### Chimeric VLP containing chimeric VP2 fusion proteins and other Birnavirus derived proteins.

Furthermore, as exemplified in Examples 10 to 12, VP2 fusion protein incorporating the insertion or substitution of one or more particular peptides of interest may also be expressed simultaneous with other IBDV or Birnaviridae proteins to favour the formation of T=13 VLP. Formation of T=13 VLP, compared to T=1 VLP, increases the copy number of VP2 proteins per VLP and may result in improved VLP stability and/or may be a preferred form for presentation or incorporation of peptides of interest. Recombinant T=13 VLP can either be generated by the expression of the pVP2-VP4-VP3 polyproteins, or by co-expression of the pVP2 and VP3 gene.

The expression of pVP2-VP4-VP3 polyprotein results in the generation of the individual proteins pVP2, VP4 and VP3 through the proteolytic activity of VP4. Expression of wild-type IBDV or Birnaviridae polyproteins usually gives rise to tubular structures containing pVP2. However, fusion of an exogenous sequence at the C-terminus of VP3, such as GFP, or the deletion of C- terminal VP3 residues strongly promote pVP2 processing and the self assembly of T=13 VLP. As exemplified in Example 10, T=13 VLP incorporating a chimeric VP2 insertion fusion protein pVP2(X)-VP4-VP3-Y containing the example peptide of interest (X or Y), cMyc (SEQ. ID. NO: 3) or Flag (SEQ. ID. NO: 2), and also VP3-Flag or VP3-cMyc, may be efficiently formed by the expression in baculovirus expression systems. Furthermore the VP3 component of the polyprotein can also be made to contain peptides of interest as exemplified by the terminal fusion of example peptides of interest, cMyc (SEQ. ID. NO: 3) and Flag (SEQ. ID. NO: 2).

Alternatively, co-expression of pVP2 and VP3 from independent gene constructs also provide the means for the formation of T=13 VLP. Furthermore, the expressions of pVP2 and VP3 fused at its N- terminus to several Histidine residues result in T=13 VLP which, in contrast to polyprotein expression systems, can be made to contains unprocessed pVP2. As shown in Example 11, co-expression in yeast of chimeric VP2 fusion proteins incorporating Flag (SEQ. ID. NO: 2) and cMyc (SEQ. ID. NO: 3) as example insertions at P loop regions, and His-VP3 incorporating Flag and cMyc as an example terminal fusion, results in efficient formation of T=13 VLP. Furthermore, the incorporation of multiple lysine (K) residues within the VP2 P loop regions also results in acceptable T=13 VLP formation. Incorporation of said K residues is aimed at the chemical conjugation to T=13 VLP of multiple copies of biological and chemical entities in a similar fashion as that described herein for T=1 VLP. Similarly, as shown in Example 12, T=13 VLP incorporating a chimeric VP2 fusion protein containing as insertion an example peptide of interest cMyc (SEQ. ID. NO: 3) or Flag (SEQ. ID. NO: 2), and His-VP3 which is C-terminally fused to same or different peptide of interest, may also be efficiently formed by the expression in baculovirus expression systems.

Therefore, another object of the present disclosure refers to T=13 VLP that result from the assembly of chimeric VP2 fusion proteins, resulting from the insertion or substitution with one or more particular peptides of interest within VP2, and VP3 proteins that may, or may not, incorporate the same or other peptides of interest. The present invention also incorporates DNA expression vectors and constructs incorporating pVP2(X)-VP4-VP3-Y polyprotein, where X an Y represent a particular DNA of interest, or alternatively DNA expression vectors that permit the simultaneous expression of chimeric VP2 fusion proteins incorporating peptides of interest and VP3 proteins or fusion proteins thereof.

### Screening of pre-selected VP2 DNA vectors for preferred insertion and/or substitution sites for particular peptides of interest.

Preferred insertion or substitution sites within VP2, judged by the ability to form VLP efficiently, may vary for different peptides of interest. Therefore, another object of the present disclosure demonstrated in Examples 13 and 14 incorporates screening methods to identify preferred insertion sites for given peptides of interest using a pre-selected panel of VP2 insertion and/or substitution DNA vectors. The screening methods can be generally conducted as follows:
1. Selection of VP2 vectors: Whereby a selection panel of VP2 DNA insertion and/or substitution vectors is made to incorporate a cloning site, preferably a multiple cloning site, which favours directional cloning of the DNA of interest.
2. Cloning of DNA of interest: Whereby the DNA of interest is cloned following standard molecular biology procedures into the previously selected panel of VP2 insertion and/or substitution vectors. DNA vectors may be arranged as an array of pre-selected vectors, one vector per well as shown in Example 13, or in a pool format containing more than one pre-selected vector per cloning reaction as shown in Example 14. The latter resulting in VP2 insertion and/or substitution libraries.
3. Transformation and expression: The resulting ligations or VP2 insertion and/or substitution libraries obtained in the previous step are transformed into suitable, bacterial, yeast, or other suitable expression systems for the evaluation of VLP formation efficiency of the individual clones. This may be carried out in individual wells for array formats, or may be carried out as a pool followed by plating and colony selection.
4. Evaluation of VLP formation capacity: For individual clones resulting from the array screen, host cells are lysed and analysed for VLP expression in each of the individual wells or spots by means of a VLP-ELISA. For pooled transformations, resulting plated colonies can be assessed for VLP production efficiency by means of a colony immunoblot assay using VP2 VLP specific antibodies against replica filters made to contain samples for each of the colonies. The chimeric VP2 fusion proteins with highest VLP formation efficiencies are selected for the confirmation and identification of the preferred insertion site. While VLP formation efficiencies above 20% are recognised as compatible with VLP formation, VLP formation efficiencies above 50%, and more preferably above 70%, are recognised as a desirable feature for resulting chimeric VP2 fusion proteins.
5. Confirmation or identification of insertion site: DNA sequencing techniques provide the means for confirmation or, for pooled transformations, the identification of the site of insertion of the DNA of interest. Furthermore VLP formation can be confirmed by electron microscopy (EM).

These screening methods against pre-selected VP2 DNA vectors permit the rapid selection of the best pre-selected sites within VP2 for the incorporation, either as insertions or substitutions, of a particular peptide of interest resulting in VLP production. These screening methods can be carried out either in an array format or in a pool format to generate libraries that can then be screened for colonies that are expressing chimeric VP2 fusion proteins that result in efficient VLP formation.

The present disclosure therefore incorporates screening methods that involve array arrangements of VP2 DNA vectors for the insertion at pre-selected points of a particular DNA of interest, and the VP2 fusion proteins resulting from the expression of the resulting chimeric VP2 fusion DNA expression vectors. Furthermore, the present disclosure also incorporates screening methods that involve the use of VP2 DNA vector pools for the insertion of DNA of interest at pre-selected points, and subsequent selection of chimeric VP2 fusion protein expression vectors which result in efficient VLP formation. Said screening methods either in an array format or in a pool format, can be carried out not only against VP2 452 but also against VP2 of different lengths or previously made to contain insertions or substitutions with the same or different peptides of interest.

### Random screening for preferred insertion and/or substitution sites for particular peptides of interest.

Preferred insertion or substitution sites within VP2, judged by the ability to form VLP efficiently, may vary for different peptides of interest. Therefore, another object of the present disclosure demonstrated in Example 15 incorporates random screening methods to identify preferred insertion sites for a given peptide of interest using a random VP2 insertion and/or substitution library for the incorporation of peptides of interest. The random screening methods can be generally conducted as follows:
1. Generation of random insertions: Whereby any transposon containing appropriate cloning site is made to randomly insert throughout the entire VP2 gene to produce a transposon insertion library. The incorporation of one, or preferably two, unique restriction enzyme sites within the randomly inserted transposon derived sequences facilitates directional cloning of the desired DNA of interest.
2. Cloning of DNA of interest: Whereby the DNA of interest is cloned into the previously generated transposon insertion library following standard molecular biology procedures.
3. Transformation and expression: The resulting ligation library is then transformed into suitable, bacterial, yeast, or other suitable expression systems for the evaluation of VLP formation efficiency of the individual colonies.
4. Evaluation of VLP formation capacity: Colonies can be assessed for VLP production efficiency by means of a colony immunoblot assay using VP2 VLP specific antibodies against replica filters made to contain samples for each of the colonies. The chimeric VP2 fusion proteins with highest VLP formation efficiencies are selected for the confirmation and identification of the preferred insertion site. While VLP formation efficiencies above 20% are recognised as compatible with VLP formation, VLP formation efficiencies above 50%, and more preferably above 70%, are recognised as a desirable feature for resulting chimeric VP2 fusion proteins.
5. Confirmation or identification of insertion site: DNA sequencing techniques provide the means for identification of the site of insertion of the DNA of interest. Furthermore VLP formation can be confirmed by electron microscopy (EM).

These random screening methods permit the rapid selection of the best pre-selected sites within VP2 for the incorporation of a particular peptide of interest resulting in VLP production. The present disclosure therefore incorporates random screening methods for the identification of preferred VP2 insertion sites for a particular DNA of interest, and the chimeric VP2 fusion proteins, and chimeric VP2 VLP, resulting from the expression of the resulting chimeric VP2 fusion protein DNA expression vectors. Furthermore, said screening methods can be carried out not only against VP2 452 but also against VP2 of different lengths or already made to contain insertions or substitutions with peptides of interest.

Therefore, in view of the above, a first aspect of the present invention refers to a fusion protein, capable of forming a Virus Like Particle, consisting of the incorporation at locations other than N- or C- terminus of one or more amino acid sequences relevant to human or veterinary health, other than an Infectious Bursal Disease Virus sequence, within a Birnaviridae virus VP2 protein, wherein preferably the VP2 protein is the VP2 protein of the Infectious Bursal Disease Virus or a VP2 that shares at least 30% amino acid sequence homology with the VP2 protein of the Infectious Bursal Disease Virus, and wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins.

In a preferred embodiment, the VP2 protein is a full length 512 amino acid pVP2, or 456, or 452 or 441 amino acid fragment thereof, or comprises at least 400 amino acids of VP2.

In another preferred embodiment, the amino acid sequences relevant to human or veterinary health are incorporated at one or more sites within the VP2 protein within the VP2 P loop regions BC (Q219-G224), DE (R249-G254), FG (T283-D287) and HI (S315-Q324), and/or outside said VP2 P loop regions, wherein the amino acid sequences relevant to human or veterinary health are the same or different for each incorporation.

In another preferred embodiment, the incorporation of amino acid sequences relevant to human or veterinary health consists in insertions.

In another preferred embodiment, the fusion protein is terminally fused either at its carboxy (C-) or amino (N-) terminal region, to a second amino acid sequence relevant to human or veterinary health which may be the same or different to the amino acid sequences relevant to human or veterinary health inserted in the P loop region or outside said P loop regions, wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins.

Another aspect of the invention refers to a Virus Like Particle constituted by assembly of the fusion protein as defined above. In a preferred embodiment of this aspect of the invention, this Virus Like Particle is constituted by assembly of the fusion protein as defined above and VP3 protein, wherein the VP3 protein is terminally fused, either at N- or C-terminus, to an amino acid sequence relevant to human or veterinary health which may be the same or different to those incorporated in VP2, wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins. In another preferred embodiment, this Virus Like Particle is obtained by the co-expression of pVP2 and VP3 from independent gene constructs or by the expression of constructs comprising the pVP2-VP4-VP3 polyprotein.

Another aspect of the invention refers to a nucleic acid encoding the fusion proteins defined above. Another aspect of the invention refers to a gene construct comprising this nucleic acid. Another aspect of the invention refers to an expression system comprising this gene construct operatively bound to transcription, and optionally translation, control elements. Another aspect of the invention refers to a host cell containing this nucleic acid or this gene construct or this expression system.

Another aspect of the invention refers to the *in vitro* use of this gene expression system for producing Virus Like Particles.

Another aspect of the invention refers to the chimeric Virus Like Particles defined above for use in medicine, drug delivery, gene therapy, as targeted agents, imaging agents, in vaccination, as antitoxins, in diagnosis, or in imaging techniques applicable to human or veterinary health.

Another aspect of the invention refers to a screening method for the location of the preferred insertion sites within VP2 proteins, or fusion proteins thereof, efficiently assembling in to Virus Like Particles, which comprises:
a. Arranging, either individually or in pools, a set of any combination of DNA vectors library for Birnaviridae VP2 protein, or fusion proteins thereof, incorporating one or more vectors with preselected cloning sites or vectors with random insertion sites for the incorporation of DNA of interest leading to any of the VP2 fusion proteins defined above;
b. Contacting the set of DNA vectors of a) with a DNA of interest coding for an amino acid sequence relevant to human or veterinary health, for the incorporation of said DNA of interest, wherein the amino acid sequence relevant to human or veterinary health is selected from the list consisting of: vaccine components, antigens and epitopes, targeting sequences, binding sequences, catalytic domains, pharmacology modulators, immunostimulators, toxins and antitoxins;
c. Transfecting, transforming or infecting host cells with the DNA expression vectors resulting from b) for the expression of chimeric VP2 fusion proteins;
d. Evaluating the VLP formation efficiency of host cells from c) and selecting clones expressing chimeric Virus Like Particles with higher efficiencies;
e. Determining or confirming the preferred insertion location of the DNA of interest.
Although the foregoing invention has been described in some detail by way of illustration and example for purposes of clarity of understanding, it will be readily apparent to those of ordinary skill in the art in light of the teachings of this invention that certain changes and modifications may be made thereto within the scope of the appended claims.

### DESCRIPTION OF THE FIGURES

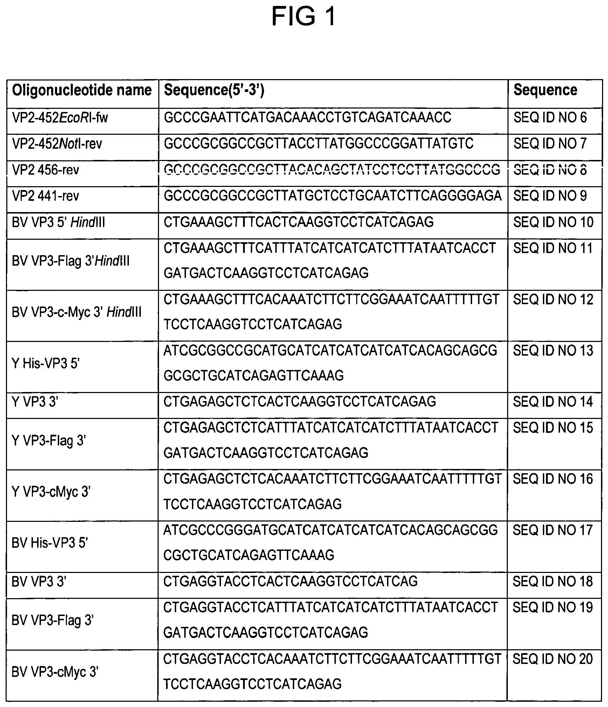

**Figure 1****: Oligonucleotides used for plasmid constructions.**
   Figure 1 describes the nucleotide sequences used as primers for PCR reactions in the construction of DNA vectors and DNA expression vectors.
**Figure 2****: Construction of SpeI-VP2 loop insertion vectors.**
   Figure 2 depicts the insertion of *Spe*I restriction sites by site directed mutagenesis generating Serine-Threonine (TS) inserts at each of the possible positions within the VP2 P loop DE region. Cloning of DNA of interest into the *Spe*I sites led the insertion of peptides of interest flanked by TS dipeptides on both ends.

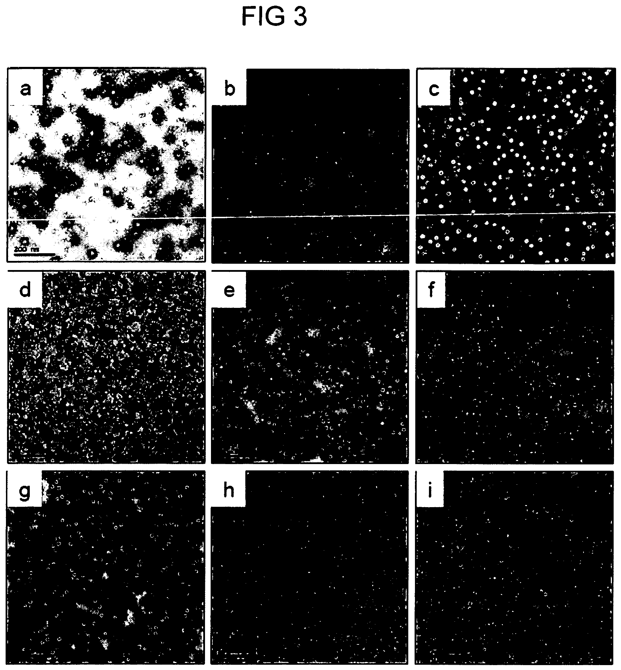

**Figure 3****: Electron microscopy analysis of purified chimeric VP2 VLP.**
   Figure 3 depicts electron micrographs (EM) of chimeric VLP obtained following DNA expression vector expression in *S*. *cerevisiae* strain 499, lysis and fractionation of soluble extracts in sucrose gradients. Panels a-i show electron microscopy (EM) pictures of purified chimeric VP2 VLP stained with uranyl actetae: (a) VP2(H₂₅₃↑Flag↑G₂₅₄), (b) VP2(H₂₅₃↑cMyc↑G₂₅₄), (c) VP2(A₃₂₁↑Flag↑ G₃₂₂), (d) VP2(A₃₂₁↑cMyc↑ G₃₂₂), (e) VP2(V₂₅₂↑Flag ↑H₂₅₃), (f) VP2(H₂₅₃↑Flag↑G₂₅₄)-Flag, (g) VP2(H₂₅₃↑cMyc↑G₂₅₄)-cMyc, (h) VP2(A₃₂₁↑Flag↑ G₃₂₂)- Flag and (i) VP2(Q₂₂₁↑K↑-H₂₅₃↑K↑-G₂₈₅↑K↑H₃₂₀↑K↑). The bar shown in the EM pictures corresponds to 200nm.

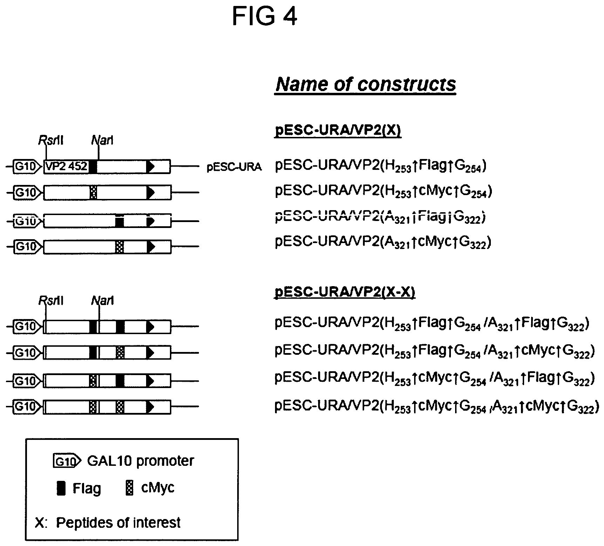

**Figure 4****: Construction of VP2 expression plasmids with multiple insertions.**
   Figura 4 depicts VP2 expression constructs with two Flag or cMyc insertions in P loop DE and HI regions pESC-URA/VP2(X-X). Double insertions result from cloning *Rsr*II and *Nar*I fragments of pESC-URANP2(H₂₅₃↑cMyc↑G₂₅₄) and pESC-URA/VP2(H₂₅₃↑Flag↑G₂₅₄) into pESC-URANP2(A₃₂₁↑cMyc↑G₃₂₂) and pESC-URA/VP2(A₂₅₃↑Flag↑G₃₂₂).

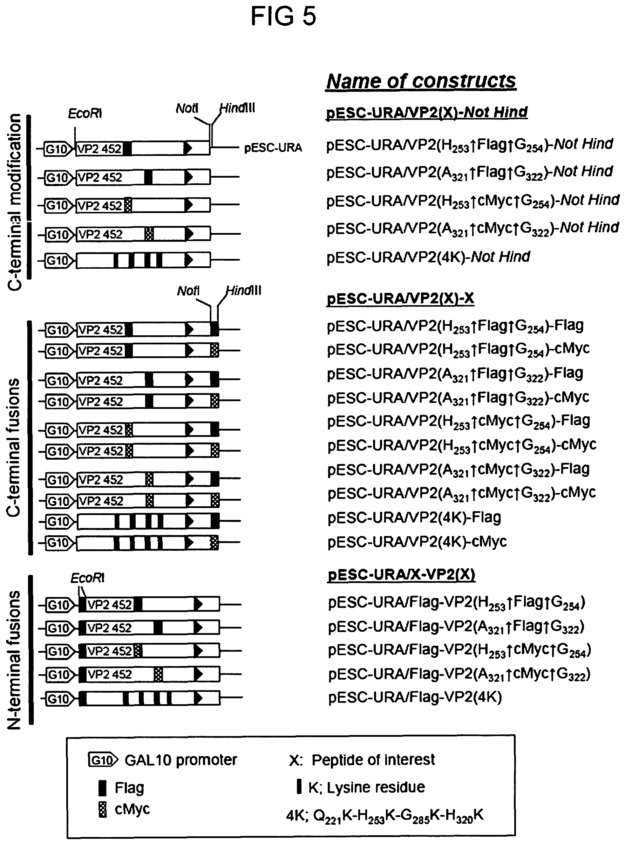

**Figure 5****: Construction of VP2 insertion and substitution constructs with N- or C-terminal fusion.**
   Figure 5 depicts the C- terminal and N- terminal fusion of example peptides of interest cMyc and Flag to chimeric VP2 fusion proteins previously made to contain cMyc, Flag or multiple Lysine substitutions. C - terminal constructs pESC-URA/VP2(X)-X were generated by insertion of Flag and cMyc peptide encoding DNA adapters downstream of the VP2 genes using restriction sites *Not*I and *Hind*III. N-terminal constructs pESC-URA/X-VP2(X) were generated by insertion at an *EcoR*I site located upstream of the VP2 start codon.

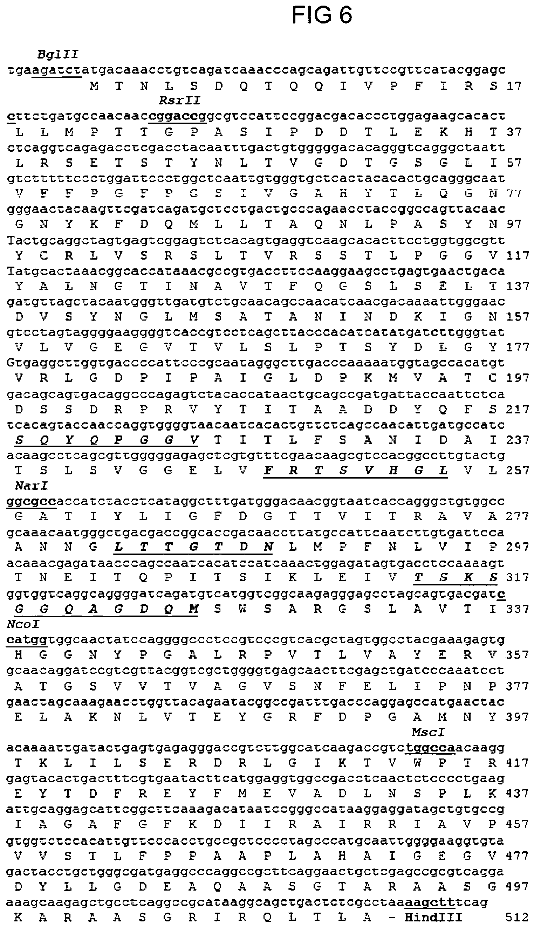

**Figure 6****: Sequence of synthetic pVP2 gene.**
   Figure 6 shows the nucleotide sequence of the synthetically produced pVP2 gene of IBDV Soroa strain (NCIB No. AAD30136) and the corresponding protein. DNA restriction sites used for cloning are underlined and named. Amino acid residues of P loop regions BC, DE, FG and HI are shown underlined.

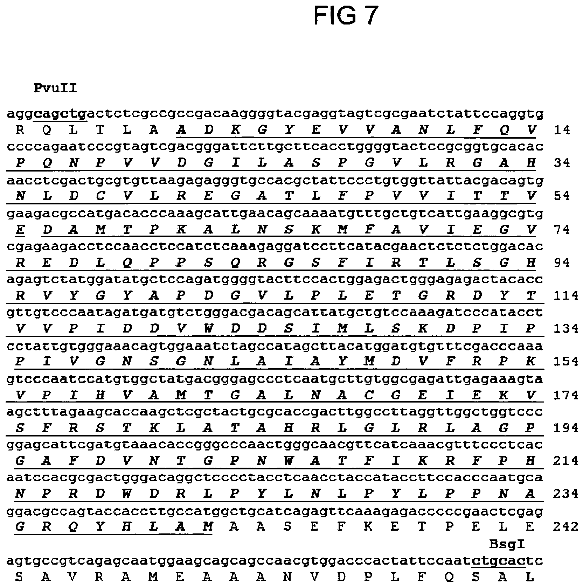

**Figure 7****: Sequence of synthetic VP4 gene.**
   Figure 7 shows the nucleotide sequence of the synthetically produced fragment of the IBDV Soroa strain segment A (NCIB No. AAD30136), which contains the 3'end of the pVP2 gene, the VP4 gene and the 5'end of the VP3 gene. The VP4 amino acid sequence is numbered and underlined and DNA restriction sites used in cloning are underlined and named.

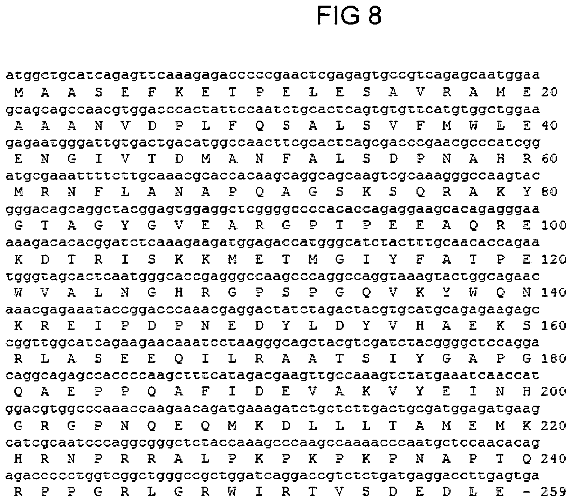

**Figure 8****: Sequence of synthetic VP3 gene.**
   Figure 8 shows the nucleotide sequence of the synthetically produced VP3 gene of IBDV Soroa strain (NCIB No. AAD30136) and the corresponding protein. Silent mutations have been introduced at positions 147 (A to T), 333 (C to T), 561 (T to C) and 600 (T to C) to mutate restriction enzyme sites *Msc*I, *Nco*I, *Hind*III and *Nco*I, respectively.

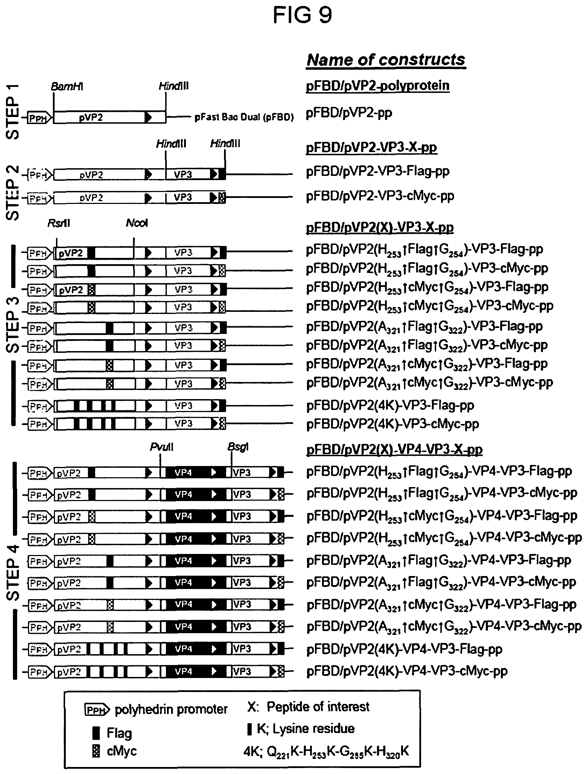

**Figure 9****: Construction of pFastBacDual pVP2-VP4-VP3-pp expression vectors.**
   Figure 9 shows the steps for the construction of pFastBacDual pVP2-VP4-VP3-pp expression vectors. Step 1: Cloning of pVP2 gene into pFastBacDual™ (pFBD) downstream of PH promoter; Step 2: insertion VP3-X genes, Flag and cMyc, at *Hind*III restriction site downstream of pVP2 gene; Step 3: Substitution of pVP2 *Rsr*II*-Nco*I fragment to generate pVP2 insertion or substitution constructs pFBD/pVP2(X)-VP3-X-pp containing Flag, c Myc or multiple lysines; Step 4: Insertion of VP4 gene at restriction sites *Pvu*II and *Bsg*I located at the 3'and 5'end of the pVP2 and VP3 gene, respectively, to generate pFBD/pVP2(X)-VP4-VP3-X-pp.

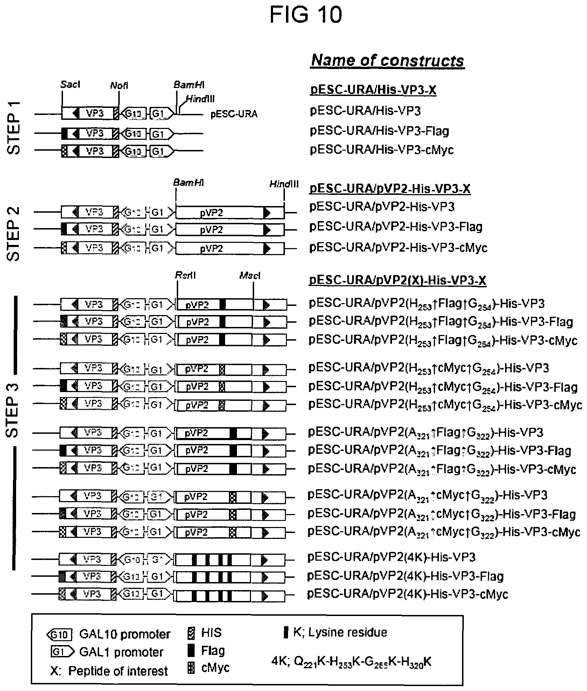

**Figure 10****: Construction of dual pESC-URA pVP2, VP3 expression vectors:**
   Figure 10 shows the steps for the construction of dual pESC-URA pVP2, VP3 expression vectors. Step 1: Cloning of His-VP3-X into pESC-URA™ downstream of G10 promoter to generate pESC-URA/His-VP3-X; Step 2: Insertion of pVP2 genes downstream of G1 promoter at a *BamH*I-*Hind*III site, generating dual pVP2-VP3 expression vectors pESC-URA/pVP2-His-VP3-X; Step 3: Substitution of pVP2 *Rsr*II-Mscl fragment to generate pVP2-VP3 insertion or substitution constructs pESC-URA/pVP2(X)-His-VP3-X.

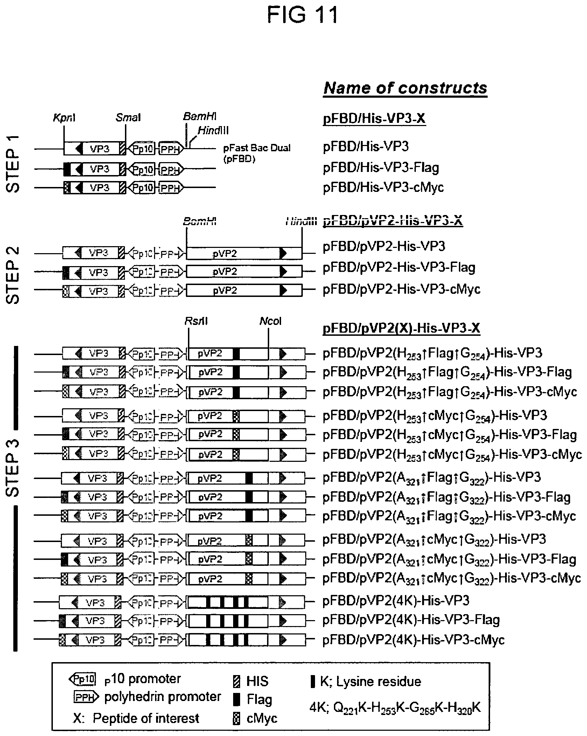

**Figure 11****: Construction of FastBacDual pVP2, VP3 expression vectors.**
   Figure 11 shows the steps for the construction of FastBacDual pVP2, VP3 expression vectors. Step 1: Cloning of His-VP3-X genes into pFastBacDual™ (pFBD) downstream of P10 promoter to generate pFBD/His-VP3-X, Step 2: Insertion of pVP2 genes downstream of PH promoter at *BamH*I-*Hind*III site, generating dual pVP2-VP3 expression vectors pFBD/pVP2-His-VP3-X; Step 3: Substitution of pVP2 *Rsr*II-*Nco*I fragment to generate pVP2-VP3 insertion or substitution constructs pFBD/pVP2(X)-His-VP3-X.

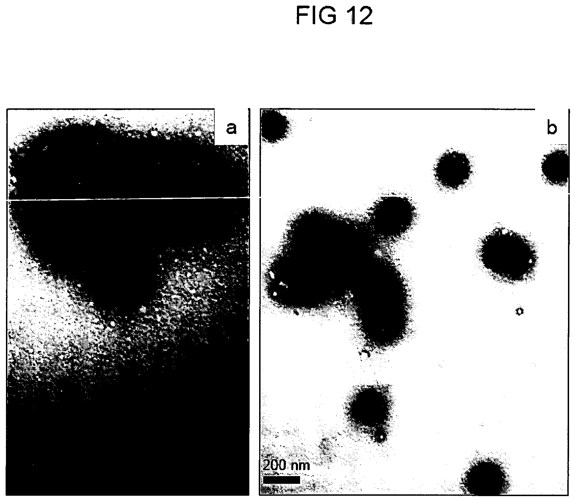

**Figure 12****: Electron microscopy (EM) analysis of purified chimeric T=13 VLP.**
   Figure 12 shows electron microscopy (EM) pictures of purified T=13 VLP samples stained with uranyl actetae following sucrose gradient purification. (1) pVP2(H₂₅₃↑Flag↑G₂₅₄)-His-VP3-Flag and (2) pVP2-His-VP3-Flag. The bar shown in the EM pictures corresponds to 200nm.

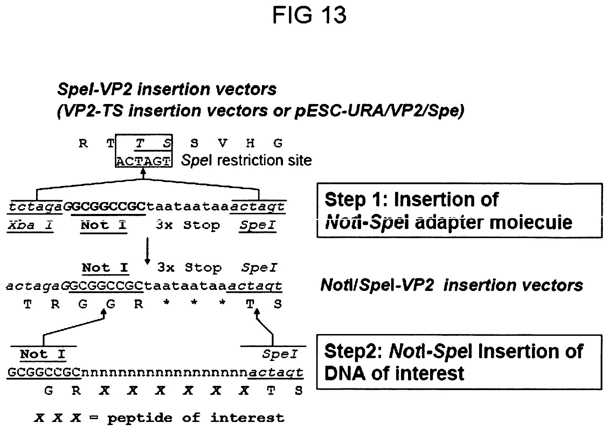

**Figure 13****: Generation of a multiple cloning site at VP2 insertion positions.**
   Figure 13 depicts the insertion of a multiple cloning site (MCS) at a *Spe*I site resulting in a *Not*I/*Spe*I cloning site containing several in-frame stop codons. Peptides of interest are introduced as *Not*I/*Spe*I insertions. Stop codons ensure that re-ligation of empty vectors during the generating of insertion libraries generate deleted versions of the VP2 protein that are unable to form VLP.

### EXAMPLES

### Example 1. Insertion of peptides of interest in P loop regions of IBDV VP2.

A collection of plasmids incorporating a *Spe*I site at each of the possible positions within the VP2 P loops, and immediately adjacent positions, was generated by site-directed mutagenesis using the yeast expression plasmid pESC-URA-VP2 452 (pESC-URA/VP2). For the construction of pESC-URA/VP2 the VP2 cDNA was amplified using oligonucleotides VP2-452*EcoR*I-fw (SEQ. ID. NO: 6) and VP2-452*Not*I-rev (SEQ. ID. NO: 7) (Figure 1) and templates pESC-URA/pVP2-512 which contains full-length IBDV VP2 512 insert (IBDV Soroa strain, NCIB No. AAD30136). The VP2 452 I nsert was coned into *EcoR*I and *Not*I digested pESC-URA(Stratagene™) in which *Spe*I site was previously deleted. The insertion of the *Spe*I restriction site generated a Serine-Threonine (TS) insertion at each of the possible positions within the VP2 P loop regions as shown in Table 1 and exemplified for P loop region DE in Figure 2. Purified *Spe*I-VP2 loop insertion vectors (VP2-TS loop insertion vectors or pESC-URA/VP2/Spe) were separately linearised by digestion with *Spe*I and ligated to DNA adapter molecules encoding for a single copy of the peptide of interest. Re-ligated plasmids were used to transform *E.coli,* and insertion clones were identified by restriction analysis and sequencing. This was carried out for each of 4 example peptides of interest, namely Flag (SEQ. ID. NO: 2), cMyc (SEQ. ID. NO: 3), V5 (SEQ. ID. NO: 4) and VSV G (SEQ. ID. NO: 5) generating a panel of VP2-peptide insertion constructs with flanking TS sequences derived from the engineered *Spe*I site. Purified VP2-TS loop insertion vectors and VP2-peptide insertion constructs were used to transform *S.cerevisiae* Y449 strain. The ability to form VLP for each of the VP2-TS loop insertion vectors [e.g.: pESC-URA/VP2(H₂₅₃↑TS↑G₂₅₄)] and VP2-peptide loop insertion constructs [e.g.: pESC-URA/VP2(H₂₅₃↑Flag↑G₂₅₄)] was quantitatively determined by VLP-ELISA using total yeast cell extracts (see Table 1). The VLP-ELISA assay made use of an antibody capable of recognising VP2 only when assembled in the form of a VLP. Briefly, ELISA plates were pre-coated with rabbit anti-VP2 and serial sample dilutions were added and incubated for 1 hour at RT. After washing, the plate is incubated with mouse anti-VP2 for 1 hour at RT followed by peroxidase assay development according to standard procedure. The EC50 for a given sample is determined as the sample dilution at which the VLP-ELISA results in 50% of the maximum signal obtained for that sample. VLP formation efficiency is in all cases expressed as the percentage of the EC50 value obtained for a given sample compared to the native VP2 452 control.

In order to confirm chimeric VP2 VLP formation, large scale yeast cultures were prepared, VLP were purified by means of sucrose gradient centrifugation, and the presence of VLP verified by electron microscopy (EM) as shown in Figure 3. VLP formation efficiencies below 20% of that of the native VP2 452 were considered as background (BG) and were taken to be an indication that the insertion location was not compatible with VLP formation for the tested peptides. The obtained results served to identify insertion sites specifically favoured by the different tested peptides of interest.

As demonstrated in Table 1 VLP formation efficiency of inserts at the BC, DE, FG and HI loops of the VP2 P domain depends on the sequence of the inserted DNA of interest and the insertion location. As an example, insertion locations, indicated by an arrow, which resulted in high VLP formation efficiency for the TS di-peptide included positions G₂₅₄↑L₂₅₅, D₂₈₇↑N₂₈₈, S₃₁₅↑K₃₁₆, K₃₁₆↑S₃₁₇, G₃₂₂↑D₃₂₃ and D₃₂₃↑Q₃₂₄, while the preferred sites for Flag insertion were H₂₅₃↑G₂₅₄, S₃₁₅↑K₃₁₆ and G₃₁₈↑G₃₁₉, for cMyc insertion D₃₂₃↑Q₃₂₄, for V5 insertion G₃₁₈↑G₃₁₉ and Q₃₂₄↑M₃₂₅, and for VSV-G insertion the G₃₁₈↑G₃₁₉ position.

**Table 1: Insertion positions in P loop regions BC, DE, FG and HI.**

| (%VLP: VLP formation efficiency; BG: Background expression; NT: Not Tested). | | | | | | |
|---|---|---|---|---|---|---|
| P-LOOP REGION | INSERT POSITION (P-loop region sequences underlined) | % VLP | | | | |
| | | TS | Flag | cMyc | V5 | VSV-G |
| **B-C** | ↑S₂₁₈**Q₂₁₉Y₂₂₀Q₂₂₁P₂₂₂G₂₂₃G₂₂₄** V₂₂₅ | 22 | BG | BG | BG | BG |
| | S₂₁₈↑**Q₂₁₉Y₂₂₀Q₂₂₁P₂₂₂G₂₂₃G₂₂₄** V₂₂₅ | 48 | 35 | BG | 25 | BG |
| | S₂₁₈**Q₂₁₉** ↑**Y₂₂₀Q₂₂₁P₂₂₂G₂₂₃G₂₂₄** V₂₂₅ | 23 | BG | 36 | 80 | 84 |
| | S₂₁₈**Q₂₁₉Y₂₂₀**↑**Q₂₂₁P₂₂₂G₂₂₃G₂₂₄** V₂₂₅ | 47 | 34 | 61 | 33 | 82 |
| | S₂₁₈**Q₂₁₉Y₂₂₀Q₂₂₁**↑**P₂₂₂G₂₂₃G₂₂₄** V₂₂₅ | 37 | 31 | BG | BG | 20 |
| | S₂₁₈**Q₂₁₉Y₂₂₀Q₂₂₁P₂₂₂**↑**G₂₂₃G₂₂₄** V₂₂₅ | 65 | 46 | 34 | 71 | 75 |
| | S₂₁₈**Q₂₁₉Y₂₂₀Q₂₂₁P₂₂₂G₂₂₃** ↑**G₂₂₄** V₂₂₅ | BG | BG | 30 | BG | BG |
| | S₂₁₈**Q₂₁₉Y₂₂₀Q₂₂₁P₂₂₂ G₂₂₃ G₂₂₄** ↑V₂₂₅ | 34 | 23 | BG | BG | 25 |
| | S₂₁₈**Q₂₁₉Y₂₂₀Q₂₂₁P₂₂₂G₂₂₃ G₂₂₄** V₂₂₅↑ | BG | BG | BG | BG | BG |
| **D-E** | ↑F₂₄₈**R₂₄₉T₂₅₀S₂₅₁V₂₅₂H₂₅₃G₂₅₄**L₂₅₅ | BG | NT | NT | NT | NT |
| | F₂₄₈↑**R₂₄₉T₂₅₀S₂₅₁V₂₅₂H₂₅₃G₂₅₄**L₂₅₅ | BG | NT | NT | NT | NT |
| | F₂₄₈**R₂₄₉**↑**T₂₅₀S₂₅₁V₂₅₂H₂₅₃G₂₅₄**L₂₅₅ | BG | BG | BG | BG | BG |
| | F₂₄₈**R₂₄₉T₂₅₀**↑**S₂₅₁V₂₅₂H₂₅₃G₂₅₄**L₂₅₅ | 43 | 37 | BG | BG | 23 |
| | F₂₄₈**R₂₄₉T₂₅₀S₂₅₁**↑**V₂₅₂H₂₅₃G₂₅₄**L₂₅₅ | 38 | BG | BG | BG | BG |
| | F₂₄₈**R₂₄₉T₂₅₀S₂₅₁V₂₅₂**↑**H₂₅₃G₂₅₄**L₂₅₅ | 26 | 66 | BG | 25 | 24 |
| | F₂₄₈**R₂₄₉T₂₅₀S₂₅₁V₂₅₂H₂₅₃**↑**G₂₅₄**L₂₅₅ | 69 | 100 | 84 | NT | 49 |
| | F₂₄₈**R₂₄₉T₂₅₀S₂₅₁V₂₅₂H₂₅₃G₂₅₄**↑L₂₅₅ | 100 | 28 | BG | BG | BG |
| | F₂₄₈**R₂₄₉T₂₅₀S₂₅₁V₂₅₂H₂₅₃G₂₅₄**L₂₅₅↑ | 35 | 25 | 22 | BG | NT |
| **F-G** | ↑L₂₈₄**T₂₈₃T₂₈₄G₂₈₅T₂₈₆D₂₈₇**N₂₈₈ | 25 | BG | NT | NT | NT |
| | L₂₈₄↑**T₂₈₃T₂₈₄G₂₈₅T₂₈₆D₂₈₇**N₂₈₈ | 62 | 45 | 34 | 23 | BG |
| | L₂₈₄**T₂₈₃**↑**T₂₈₄G₂₈₅T₂₈₆D₂₈₇** N₂₈₈ | 79 | 45 | 23 | 95 | 80 |
| | L₂₈₄**T₂₈₃T₂₈₄**↑**G₂₈₅T₂₈₆D₂₈₇** N₂₈₈ | 81 | 56 | 45 | 68 | 70 |
| | L₂₈₄**T₂₈₃T₂₈₄G₂₈₅**↑**T₂₈₆D₂₈₇** N₂₈₈ | 85 | 75 | NT | NT | NT |
| | L₂₈₄**T₂₈₃T₂₈₄G₂₈₅T₂₈₆**↑**D₂₈₇** N₂₈₈ | 90 | 66 | 32 | NT | 57 |
| | L₂₈₄**T₂₈₃T₂₈₄G₂₈₅T₂₈₆D₂₈₇**↑ N₂₈₈ | 97 | 62 | NT | NT | NT |
| | L₂₈₄**T₂₈₃T₂₈₄G₂₈₅T₂₈₆D₂₈₇**N₂₈₈↑ | 44 | NT | NT | NT | NT |
| **H-I** | ↑T₃₁₄**S₃₁₅K₃₁₆S₃₁₇G₃₁₈G₃₁₉Q₃₂₀A₃₂₁G₃₂₂D_{3 23}Q₃₂₄**M₃₂₅ | 34 | NT | NT | NT | NT |
| | T₃₁₄↑**S₃₁₅K₃₁₆S₃₁₇G₃₁₈G₃₁₉Q₃₂₀A₃₂₁G₃₂₂D_{3 23}Q₃₂₄**M₃₂₅ | 71 | 58 | 65 | NT | 83 |
| | T₃₁₄**S₃₁₅**↑**K₃₁₆S₃₁₇G₃₁₈G₃₁₉Q₃₂₀A₃₂₁G₃₂₂D_{3 23}Q₃₂₄**M₃₂₅ | 100 | 100 | NT | 38 | 36 |
| | T₃₁₄**S₃₁₅K₃₁₆**↑**S₃₁₇G₃₁₈G₃₁₉Q₃₂₀A₃₂₁G₃₂₂D_{3 23}Q₃₂₄**M₃₂₅ | 100 | NT | NT | NT | NT |
| | T₃₁₄**S₃₁₅K₃₁₆S₃₁₇**↑**G₃₁₈G₃₁₉Q₃₂₀A₃₂₁G₃₂₂D₃** | 59 | 85 | NT | NT | NT |
| | **₂₃Q₃₂₄**M₃₂₅ | | | | | |
| | T₃₁₄**S₃₁₅K₃₁₆S₃₁₇G₃₁₈**↑**G₃₁₉Q₃₂₀A₃₂₁G₃₂₂D_{3 23}Q₃₂₄**M₃₂₅ | 56 | 100 | 36 | 97 | 100 |
| | T₃₁₄**S₃₁₅K₃₁₆S₃₁₇G₃₁₈G₃₁₉**↑**Q₃₂₀A₃₂₁G₃₂₂D_{3 23}Q₃₂₄**M₃₂₅ | 83 | NT | NT | 77 | 79 |
| | T₃₁₄**S₃₁₅K₃₁₆S₃₁₇G₃₁₈G₃₁₉Q₃₂₀↑A₃₂₁G₃₂₂D_{3 23}Q₃₂₄**M₃₂₅ | 91 | 66 | 81 | NT | NT |
| | T₃₁₄**S₃₁₅K₃₁₆S₃₁₇G₃₁₈G₃₁Q₃₂₀A₃₂₁↑G₃₂₂D_{3 23}Q₃₂₄**M₃₂₅ | 73 | 62 | 67 | 34 | 33 |
| | T₃₁₄**S₃₁₅K₃₁₆S₃₁₇G₃₁₈G₃₁₉Q₃₂₀A₃₂₁G₃₂₂↑D_{3 23}Q₃₂₄**M₃₂₅ | 100 | 60 | NT | 72 | 58 |
| | T₃₁₄**S₃₁₅K₃₁₆S₃₁₇G₃₁₈G₃₁₉Q₃₂₀A₃₂₁G₃₂₂D_{32 3}↑Q₃₂₄**M₃₂₅ | 100 | 86 | 99 | 64 | NT |
| | T₃₁₄**S₃₁₅K₃₁₆S₃₁₇G₃₁₈G₃₁₉Q₃₂₀A₃₂₁G₃₂₂D_{32 3}Q₃₂₄**↑M₃₂₅ | 75 | 70 | NT | 95 | NT |
| | T₃₁₄**S₃₁₅K₃₁₆S₃₁₇G₃₁₈G₃₁₉Q₃₂₀A₃₂₁G₃₂₂D_{32 3}Q₃₂₄**M₃₂₅↑ | BG | BG | NT | NT | NT |

### Example 2. Insertion of peptides of interest in P loop regions of VP2 of different lengths.

To test if the length of VP2 proteins could affect VLP formation efficiency, IBDV VP2 of different lengths at their C-terminal, namely VP2 with 452, 441 and 456 amino acids (VP2 452, VP2 441 and VP2 456), were compared for their capacity to incorporate insertions of peptides of interest, namely cMyc (SEQ. ID. NO: 3) and Flag (SEQ. ID. NO: 2), in locations within P loop regions DE and HI. For the construction of pESC-URA/VP2 456 and pESC-URA/VP2 441 the VP2 cDNA was amplified using oligonucleotides VP2 452*EcoR*I-fw (SEQ. ID. NO: 6) and VP2 456-rev (SEQ. ID. NO: 8) or VP2 441-rev (SEQ. ID. NO: 9) and template pESC-URA/pVP2 512 which contains full-length VP2 512 insert. Purified VP2 456 and VP2 441 gene fragments were cloned into *EcoR*I and *Not*I digested plasmids pESC-URA/VP2 452. For this purpose VP2 loop insertion plasmids [e.g.: pESC-URA/VP2 (H₂₅₃↑Flag↑G₂₅₄), were digested with *Rsr*II and *Msc*I restriction enzymes, which cut within the VP2 gene at amino acid position G₂₄-W₄₁₄, and the purified VP2 gene fragments were cloned into *Rsr*II and *Msc*I digested plasmids pESC-URA/VP2 441 and pESC-URA/VP2 456. Correct clones were identified by restriction analysis and sequencing and transformed into *S.cerevisiae* Y449 strain to evaluate VP2 expression and VLP formation efficiency by quantitative VLP-ELISA using total yeast cell extracts.

As demonstrated in Table 2, VLP production efficiency varied according to the insertion site within VP2 as well as with VP2 length. Furthermore, it was observed that shorter VP2 in some cases resulted in improved VLP formation efficiency.

**Table 2: VLP formation of VP2 constructs with insertion of peptides of interest in loop regions of VP2 441 and VP2 456.**

| (Peptides of interest appear underlined and flanked by TS linker sequences; BG: Background expression; %VLP: VLP formation efficiency). | | | |
|---|---|---|---|
| P-LOOP REGION | INSERTION POSITION (INSERTED PEPTIDE SEQUENCE) | VP2 PROTEIN LENGTH | %VLP |
| D-E | H₂₅₃↑Flag↑G₂₅₄ (TS**DYKDDDDKGSGGSSDYKDDDDKS**TS) | VP2 452 | 100 |
| | | VP2 441 | 100 |
| | | VP2 456 | 56 |
| H-I | A₃₂₁↑Flag↑ G₃₂₂ (TS**DYKDDDDKGSGGSSDYKDDDDKS**TS) | VP2 452 | 62 |
| | | VP2 441 | 67 |
| | | VP2 456 | 56 |
| D-E | S₂₅₁↑Flag↑V₂₅₂ (TS**DYKDDDDKGSGGSSDYKDDDDKS**TS) | VP2 452 | BG |
| | | VP2 441 | 34 |
| | | VP2 456 | BG |
| D-E | H₂₅₃↑cMyc↑G₂₅₄ (TS**EQKLISEEDLS**TS) | VP2 452 | 84 |
| | | VP2 441 | 83 |
| | | VP2 456 | 54 |

### Example 3: Substitution of P loop regions for peptides of interest.

To facilitate substitution of P loop regions by peptides of interest, cloning vectors were generated in which the codons within the P loop regions were replaced by a sequence encoding for a short linker containing a *Not*I restriction enzyme site using a pESC-URA/VP2 452 plasmid with a mutation in the *Not*I site downstream of the VP2 452 gene (pESC-URA/VP2 452 [Δ*Not*I]). In one series of mutants the entire P loop region was deleted and in another series all except the first and last codon of each P loop were deleted, as shown in Table 3. Example peptides of interest, namely Flag (SEQ. ID. NO: 2) and cMyc (SEQ. ID. NO: 3) were cloned following standard procedures as *Not*I dsDNA fragments for the DNA of interest into *Not*I linearised cloning vectors [e.g.: pESC-URANP2/ΔY₂₂₀-G₂₂₃] generating in frame insertions. Purified constructs [e.g.: pESC-URAA/P2/ΔY₂₂₀-G₂₂₃/Q₂₁₉↑Flag↑G₂₂₄ were used to transform *S.cerevisiae* Y449 and the VLP production efficiency, shown in Table 3, was quantitatively determined by VLP-ELISA on total yeast cell extracts. Table 3 shows VLP formation efficiency when VP2 loop sequences are substituted with peptides of interest. As demonstrated in Table 3, VLP formation efficiency was generally low, but still equal or above 20%, and varied according to the site of substitution within VP2. Furthermore, it was observed that retention of the first and last residue of the P loop regions improved VLP formation efficiency.

**Table 3: Substitutions of VP2 loop sequences with Peptide of Interest.**

| (Peptides of interest appear underlined and flanked by linker sequences; BG Background expression; %VLP: VLP Formation Efficiency). | | | |
|---|---|---|---|
| P-LOOP REGION | SUBSTITUTION POSITION | INSERTED PEPTIDE (SEQUENCE) | %VLP |
| B-C | S₂₁₈↑...↑V₂₂₅ | Flag (GGSGR**DYKDDDDKGSGGSSDYKDDDDK**GGSGR) | BG |
| | | cMyc (GGSGR**EQKLISEEDL**GGSGR) | BG |
| | S₂₁₈↑Q₂₁₉↑...↑G₂₂₄V₂₂₅ | Flag (GGSGR**DYKDDDDKGSGGSSDYKDDDDK**GGSGR) | 24 |
| | | cMyc (GGSGR**EQKLISEEDL**GGSGR) | 33 |
| D-E | F₂₄₈↑...↑L₂₅₅ | Flag (GGSGR**DYKDDDDKGSGGSSDYKDDDDK**GGSGR) | 25 |
| | | cMyc (GGSGR**EQKLISEEDL**GGSGR) | BG |
| | F₂₄₈R₂₄₉↑...↑G₂₅₄L₂₅₅ | Flag (GGSGR**DYKDDDDKGSGGSSDYKDDDDK**GGSGR) | 35 |
| | | cMyc (GGSGR**EQKLISEEDL**GGSGR) | 27 |
| F-G | L₂₈₄↑...↑N₂₈₈ | Flag (GGSGR**DYKDDDDKGSGGSSDYKDDDDK**GGSGR) | BG |
| | | cMyc (GGSGR**EQKLISEEDL**GGSGR) | BG |
| | L₂₈₄T₂₈₃↑...↑D₂₈₇N₂₈₈ | Flag (GGSGR**DYKDDDDKGSGGSSDYKDDDDK**GGSGR) | 22 |
| | | cMyc (GGSGR**EQKLISEEDL**GGSGR) | 21 |
| H-I | T₃₁₄↑...↑M₃₂₅ | Flag (GGSGR**DYKDDDDKGSGGSSDYKDDDDK**GGSGR) | BG |
| | | cMyc (GGSGR**EQKLISEEDL**GGSGR) | 25 |
| | T₃₁₄S₃₁₅↑...↑Q₃₂₄M₃₂₅ | Flag (GGSGR**DYKDDDDKGSGGSSDYKDDDDK**GGSGR) | 28 |
| | | cMyc (GGSGR**EQKLISEEDL**GGSGR) | 34 |

### Reference Example 4: Incorporation of lysine (K) residues in loop regions and chemical conjugation of cMyc.

To generate additional conjugation sites within VP2, lysine (K) residues were cloned as substitutions of P loop region residues. Briefly, different lysine mutants were generated by site-directed mutagenesis of plasmid pESC-URA/VP2 [e.g.: pESC-URA/VP2(Q₂₂₁K-H₂₅₃K-G₂₈₅K); mutations are expressed as the mutated amino acid followed by the induced K residue]. Purified constructs were transformed into *S.cerevisiae* Y449 and VP2 expression and VLP formation efficiency was determined by quantitative VLP-ELISA on total yeast cell extracts. Table 4 shows VLP production efficiency of mutants expressed as % VLP production efficiency compared the native VP2-VLP.

As shown in Table 4, mutants with 2, 3 and 4 additional lysine residues were expressed to high levels and T=1 VLP production efficiency was comparable to that of native VP2 452. Only mutant VP2(G₂₈₅K-Q₃₂₀K) made to contain a lysine residue in replacement G₂₈₅ and another in replacement of Q₃₂₀ did not form VLP. EM analysis of the K substitution constructs confirmed the presence of T=1 VLP. For the chemical conjugation VLP were purified by means of sucrose gradient centrifugation and the peptide conjugation efficiency of the native VP2 452, 3K and 4K mutant VLP were compared. VLP-peptide conjugates were prepared with 0.5-2 mg of purified VP2 VLP samples. In a first step, VLP were incubated with 3-maleimidobenzoic acid N-succinimidyl ester at a ratio of 1:50 for 30 minutes at 20°C and subsequently dialyzed to eliminate MBS. The resulting VLP-MBS and the example peptide of interest, namely a modified cMyc (SEQ. ID. NO: 3) made to contain a terminal cysteine (C) residue, were mixed 1:50 (VLP-MBS: cMyc) and incubated over night at 4°C, pH 7.0. The final conjugation (VLP - cMyc) product was dialysed, freeze dried and the amount of conjugated peptide was quantified by ELISA using cMyc specific antibodies. Results showed clearly that the incorporation of additional K residues by means of substitutions increased conjugation efficiency to peptides of interest.

**Table 4: Chimeric VP2 VLP incorporating Lysine substitutions in P loop regions.**

| (%VLP: VLP formation efficiency; BG: Background expression; NT: Not Tested). | | | | | | |
|---|---|---|---|---|---|---|
| NUMBER OF INCORPORATED K RESIDUES | B-C Q₂₂₁K | D-E H₂₅₃K | F-G G₂₈₅K | H-I Q₃₂₀K. | % VLP | VLP MORPHOLOGY (EM) |
| 0 | | | | | 100 | T=1 |
| 1 | | | | X | NT | NT |
| 1 | | | X | | NT | NT |
| 1 | | X | | | NT | NT |
| 1 | X | | | X | NT | NT |
| 2 | | | X | X | BG | T=1 |
| 2 | | X | | X | 66 | T=1 |
| 3 | X | X | X | | 55 | T=1 |
| 4 | X | X | X | X | 45 | T=1 |

### Example 5. Identification of VP2 insertion sites outside P loop regions by random transposon mutagenesis with Tn5.

Insertion of the Tn5 transposon results in the insertion of 57 nucleotides coding for 19 amino acids. Random insertion mutagenesis of IBDV VP2 with Tn5, using Plasmid pESC-URA/VP2 452 (Δ*Spe*I/*Not*I) and EZ-Tn5 In-Frame Linker Insertion Kit (Epicentre™), generated a library of DNA clones with insertions along the complete VP2 452 amino acid sequence. Transformation of competent cells was carried out using *Transformax EC100 Electrocompetent cells,* obtaining >200,000 clones with resistance to ampicillin (provided by the plasmid) and kanamycin (provided by the transposon). Plasmid DNA of all clones were isolated and purified to form a first Tn5 library. DNA from this first Tn5 library was digested with *Eco*RI and *Bgl*II to purify the band corresponding to the VP2 coding sequence with one Ez-Tn5 insertion. This band was cloned in the pESC-URA vector digested with *Eco*RI and *Bgl*II to generate a second library, with random insertions only in the VP2 gene. Ligation was transformed as above and 65,000 clones were obtained. Plasmid DNA of all clones were isolated and purified to form a second Tn5 library. DNA from this second Tn5 library was digested with *Not*I and re-ligated, to eliminate the kanamycin-resistance gene from the insertion. Religation product was transformed as above to obtain 350,000 new clones that constituted the final 19 amino acid random Tn5 insertion library. *S.cerevisiae* Y499 yeast cells were transformed with 10 µg of this final random Tn5 insertion library and plated into YNB/CSM-URA + 2% glucose plates. 110,000 yeast clones were obtained and were transferred to galactose-containing plates. Colonies grown in the presence of galactose were transferred to a PVDF membrane to analyze VP2 VLP expression by colony immunoblot using VP2-VLP specific antibodies. A subset of positive clones were growth individually in liquid YNB/CSM-URA + 2% galactose medium and VLP expression was analyzed by VLP-ELISA. Insertions within VP2 were identified by sequencing the PCR product obtained from each clone using VP2 and Tn5 specific primers. Tn5 mutagenesis led to the insertion of 19 amino acid peptides which consist of one of three possible 15 residue core peptides and 4 variable residues depending of the insertion site. Evaluation of the capacity of Tn5 constructs to produce VLP resulted in the identification of a number of insertion sites, listed in Table 5, compatible with VLP formation, and with potential for the generation of chimeric VP2 VLP with insertion or substitutions with peptides of interest.

The identification of the Tn5 insertion locations was carried out from limited analysis of 150 clones from the final random Tn5 insertion library. Other insertion locations may also be contained in said insertion library and also represent potential insertion or substitution sites for peptides of interest.

**Table 5. VP2 insertion sites identified by random Tn5 transposon mutagenesis.**

| (15 residue long TN5 derived core peptides are shown underlined) | |
|---|---|
| INSERTION POSITION | INSERTED PEPTIDE |
| E₃₄↑K₃₅ | T**VSCTHLAAARCVQET**ALE |
| E₃₄↑K₃₅ | L**SLVHILRPQDVYKRQ**TLE |
| E₄₁↑T₄₂ | T**VSCTHLAAARCVQET**GSE |
| T₄₄↑Y₄₅ | S**VSCTHLAAARCVQET**AST |
| G₅₅↑L₅₆ | L**SLVHILRPQDVYKRQGS**G |
| Q₇₅↑G₇₆ | A**VSCTHLAAARCVQET**ALQ |
| G₇₆↑N₇₇ | L**SLVHILRPQDVYKRQ**LQG |
| S₁₀₃↑R₁₀₄ | L**SLVHILRPQDVYKRQ**LVS |
| S₁₀₃↑R₁₀₄ | R**CLLYTSCGRKMCTRD**RVS |
| T₁₀₇↑V₁₀₈ | V**CLLYTSCGRKMCTRD**STV |
| S₁₁₁↑T₁₁₂ | T**VSCTHLAAARCVQET**GSS |
| T₁₁₂↑L₁₁₃ | P**VSCTHLAAARCVQET**AST |
| G₁₁₅↑G₁₁₆ | L**SLVHILRPQDVYKRQ**LPG |
| G₁₁₆↑V₁₁₇ | A**VSCTHLAAARCVQET**AGG |
| A₁₁₉↑L₁₂₀ | P**VSCTHLAAARCVQET**VYA |
| V₁₇₈↑R₁₇₉ | L**SLVHILRPQDVYKRQ**GYV |
| P₁₈₃↑I₁₈₄ | L**SLVHILRPQDVYKRQ**GDP |
| A₁₈₆↑I₁₈₇ | I**CLLYTSCGRKMCTRD**SPA |
| G₁₈₈↑L₁₈₉ | P**VSCTHLAAARCVQET**AIG |
| D₁₉₀↑P₁₉₁ | P**VSCTHLAAARCVQET**GLD |
| C₁₉₇↑D₁₉₈ | A**VSCTHLAAARCVQET**ATC |
| D₁₉₈↑S₁₉₉ | T**VSCTHLAAARCVQET**ACD |
| D₂₀₁↑R₂₀₂ | T**VSCTHLAAARCVQET**GSD |
| Y₂₀₆↑T₂₀₇ | T**CLLYTSCGRKMCTRD**RVY |
| S₂₄₀↑L₂₄₁ | P**VSCTHLAAARCVQET**VTS |
| N₄₃₃↑S₄₃₄ | S**VSCTHLAAARCVQET**DLN |
| N₄₃₃↑S₄₃₄ | S**CLLYTSCGRKMCTRDS**LN |
| A₄₅₀↑I₄₅₁ | I**CLLYTSCGRKMCTRD**SRA |
| R₄₅₂↑ | L**SLVHILRPQDVYKRQ**AIR |

### Example 6: Identification of VP2 insertion sites outside P loops by random transposon mutagenesis with Mu.

Insertion of Mu transposon results in the insertion of 15 nucleotides coding for a 5 amino acid peptide. The sequence of the insert varied between CGR, PRH or AAA, with two random flanking residues, depending on the insertion site and reading frame. Random insertion mutagenesis of IBDV VP2 generated a DNA library of clones with insertions along the complete VP2 452 sequence. In particular, a yeast expression plasmid pESC-URA/VP2 was used to generate a random-insertion library using the Entranceposon M1-Cam^{R}(M1-Cam) Mutation Generation System F-701 (MGS™). Reaction was made according to manufacturer instructions and 0.5-1 µl were used to transform *Transformax EC100 Electrocompetent cells,* obtaining > 100,000 clones with resistance to ampicillin (provided by the plasmid) and chloramphenicol (provided by the transposon). Plasmid DNA for all clones was simultaneously purified to form a first Mu library. This first Mu library was digested with *EcoR*I and *Bgl*II and the band corresponding to the VP2 coding sequence with one Mu insertion was isolated. This band was cloned in the pESC-URA vector digested with *EcoR*I and *Bgl*II to generate a second library, with random insertions only in the VP2 gene. Ligation was transformed into electrocompetent cells and > 80,000 clones were obtained. Plasmid DNA for all clones was simultaneously purified to form a second Mu library. DNA from the second Mu library was digested with *Not*I and re-ligated, to eliminate the chloramphenicol - resistance gene from the insertion. This DNA was transformed as described above obtaining > 100,000 new clones that constituted the final 5 amino acid random Mu insertion library. *S.cerevisiae* Y449 yeast cells were transformed with 10 µg of random Mu insertion library and plated into YNB/CSM-URA + 2% glucose plates. -110,000 yeast clones were obtained and transferred to galactose-containing plates. Colonies grown in the presence of galactose were transferred to a PVDF membrane to analyze for VP2 expression by colony immunoblot. A subset of positive clones were growth individually in liquid YNB/CSM-URA+2% galactose medium and VLP expression was analyzed by VLP-ELISA.

Insertions were identified by sequencing the PCR product obtained from each clone using VP2 and Tn5 specific primers. Mu mutagenesis led to the insertion of 5 amino acid peptides which consist of one of three possible 3 residue core peptides and 2 variable residues depending of the insertion site. Evaluation of the capacity of Mu constructs to produce VLP resulted in the identification of a number of insertion sites, listed in Table 6, compatible with VLP formation, and with potential for the generation of chimeric VP2 VLP incorporating peptides of interest by insertion or substitution.

The identification of the Mu insertion locations was carried out from limited analysis of 200 clones from the final random Mu insertion library. Other insertion locations may also be contained in said insertion library and also represent potential insertion or substitution sites for peptides of interest.

**Table 6: VP2 insertion sites identified by random Mu transposon mutagenesis.**

| (Mu derived core peptides are shown underlined) | |
|---|---|
| INSERTION POSITION | INSERTED PEPTIDE |
| M₁↑T₂ | M**RPH**M |
| G₅₀↑D₅₁ | **CGR**MG |
| Y₇₂↑T₇₃ | **CGR**NY |
| G₇₆↑N₇₇ | **AAA**QG |
| S₁₀₃↑R₁₀₄ | **CGR**MS |
| V₁₀₈↑R₁₀₉ | **AAA**TV |
| L₁₁₃↑P₁₁₄ | **RPH**TL |
| G₁₁₆↑V₁₁₇ | **CGR**SG |
| G₁₂₂↑T₁₂₃ | **CGR**NG |
| I₁₈₄↑P₁₈₅ | **CGR**TI |
| A₁₈₆↑I₁₈₇ | **AAA**PA |
| A₁₈₆↑I₁₈₇ | **CGR**TA |
| G₁₈₈↑L₁₈₉ | **CGR**IG |
| D₁₉₈↑S₁₉₉ | **AAA**CD |
| D₂₀₁↑R₂₀₂ | M**RPH**D |
| L₃₇₉↑A₃₈₀ | **RPH**EL |
| G₃₉₃↑A₃₉₄ | **AAA**PG |
| N₃₉₆↑Y₃₉₇ | **AAA**MN |
| M₄₂₇↑E₄₂₈ | D**AAA**M |
| L₄₃₆↑K₄₃₇ | **AAA**PL |
| G₄₄₀↑A₄₄₁ | **CGR**NG |
| F₄₄₄↑K₄₄₅ | **CGR**SF |
| R₄₄₉↑A₄₅₀ | V**RPH**R |

### Example 7: Cloning of Flag or cMyc outside VP2 P loop regions.

Removal of Tn5 or Mu transposons randomly inserted in VP2 genome resulted in inserts of 57 or 15 nucleotides encoding for a 19 or 5 amino acid insertions respectively. Resulting inserts contained a *Not*I restriction site that permitted cloning of DNA of interest. Resulting vectors [e.g.: pESC-URA/VP2(G₇₆↑Tn5↑N₇₇) ; Insertion location of Tn5 is indicated by the arrows] were digested with *Not*I and ligated to a DNA of interest, such as Flag (SEQ. ID. NO: 2) or cMyc (SEQ. ID. NO: 3), with *Not*I sticky ends. The resulting constructs [e.g.: pESC-URA/VP2(G₇₆↑Tn5-Flag↑N₇₇)] were transformed into *S.cerevisiae* Y449 and the ability to form VLP, shown in Table 7, was quantitatively determined by VLP-ELISA on total yeast cell extracts.

In addition, insertion vectors were also constructed by site directed mutagenesis to generate *Spe*I restriction sites at previously identified transposon insertion sites. Insertion of the *Spe*I restriction site generated Serine-Threonine (TS) insertion at the desired pre-identified points within VP2. For insertion of DNA of interest the resulting insertion vectors [e.g.: pESC-URA/VP2(G₇₆↑TS↑N₇₇)] were digested with *Spe*I and ligated to a DNA of interest, such as Flag or cMyc, *Spe*I sticky ends. Resulting constructs [e.g.: pESC-URA/VP2(G₇₆↑TS-Flag↑N₇₇)] were transformed into *S.cerevisiae* Y449 and the ability to form VLP, shown in Table 7, was quantitatively determined by VLP-ELISA on total yeast cell extracts.

The insertion of peptides of interest into the Tn5 or Mu inserts generally resulted in decreased, but still equal or above 20%, VLP formation efficiency compared to the Tn5 or Mu containing constructs. However the data demonstrated the viability of the approach in identifying potential VP2 insertion or substitution locations for peptides of interest by means of transposon mutagenesis.

**Table 7: VP2 insertions at sites outside the P loop regions.**

| (Inserted peptides of interest are shown underlined with the reminder amino acids resulting from the inserted Tn5 or Mu transposon, or the engineered TS site; BG: Background expression; %VLP: VLP formation efficiency). | | | |
|---|---|---|---|
| POSITION | Insert | INSERTED PEPTIDE | %VLP |
| Q₇₆↑N₇₇ | Tn5 | LSLVHILRPQDVYKRQLQG | 66 |
| | Tn5-cMyc | LSLVHILRP**EQKLISEEDLS**RPQDVYKRQLQG | 45 |
| | TS-cMyc | TS**EQKLISEEDL**TS | 88 |
| | Tn5-Flag | LSLVHILRP**DYKDDDDKGSGGSSDYKDDDDK**RPQDVYKRQLQG | 55 |
| | TS-Flag | TS**DYKDDDDKGSGGSSDYKDDDDK**TS | 77 |
| T₁₁₂↑L_{11 3} | Tn5 | PVSCTHLAAARCVQETAST | 56 |
| | Tn5-cMyc | PVSCTHLAA**EQKLISEEDL**SPAARCVQETAST | 22 |
| | TS-cMyc | TS**EQKLISEEDL**TS | 71 |
| | Tn5-Flag | PVSCTHLAATS**DYKDDDDKGSGGSSDYKDDDDK**TSSPAARCVQETAST | BG |
| | TS-Flag | TS**DYKDDDDKGSGGSSDYKDDDDK**TS | 80 |
| A₁₁₉↑L_{12 0} | Tn5 | PVSCTHLAAARCVQETVYA | 116 |
| | Tn5 | PVSCTHLAA**EQKLISEEDL**SPAARCVQETVYA | 33 |
| | Tn5-cMyc | TS**EQKLISEEDL**TS | 100 |
| | TS-cMyc | PVSCTHLAATS**DYKDDDDKGSGGSSDYKDDDDK**TSSPAARCVQETVYA | 25 |
| | Tn5-Flag | TS**DYKDDDDKGSGGSSDYKDDDDK**TS | 97 |
| G₁₈₈↑L_{18 9} | Tn5 | PVSCTHLAAARCVQETAIG | 37 |
| | Tn5-cMyc | PVSCTHLAA**EQKLISEEDL**SPAARCVQETAIG | BG |
| | TS-cMyc | TS**EQKLISEEDL**TS | 43 |
| | Tn5-Flag | PVSCTHLAATS**DYKDDDDKGSGGSSDYKDDDDK**TSSPAARCVQETAIG | 25 |
| | TS-Flag | TS**DYKDDDDKGSGGSSDYKDDDDK**TS | 43 |
| I₁₈₄↑P₁₈₅ | Mu | I**CGR**TIP | 65 |
| | Mu-cMyc | ICGP**TEQKLISEEDL**SGRTIP | 56 |
| | cMyc | TS**TEQKLISEEDL**STS | 63 |
| | Mu-Flag | ICGP**DYKDDDDKGSGGSSDYKDDDDK**SGRTIP | 64 |
| | Flag | TS**DYKDDDDKGSGGSSDYKDDDDK**TS | 70 |
| L₃₇₉↑A_{38 0} | Mu | L**RPH**ELA | 65 |
| | Mu-cMyc | LRP**TEQKLISEEDLS**RPHELA | 56 |
| | cMyc | TS**TEQKLISEEDLS**TS | 67 |
| | Mu-Flag | LRP**DYKDDDDKGSGGSSDYKDDDDKS**RPHELA | 56 |
| | Flag | TS**DYKDDDDKGSGGSSDYKDDDDK**TS | 63 |

### Example 8: Chimeric VP2 VLP incorporating multiple VP2 insertions.

In order to evaluate the capacity of VP2 VLP to incorporate peptide insertions at two different sites, a series of constructs were generated as shown in Figure 4. Plasmids pESC-URA/VP2(H₂₅₃↑Flag↑G₂₅₄) and pESC-URA/VP2(H₂₅₃↑cMyc↑G₂₅₄) were digested with restriction enzymes *Rsr*II and *Nar*I. The DNA fragments encoding for N-terminal part of VP2 including the insertions at H₂₅₃↑G₂₅₄ were purified and cloned into the *Rsr*II, *Nar*I digested plasmids pESC-URA/VP2(A₃₂₁↑Flag↑G₃₂₂) and pESC-URAA/P2(A₃₂₁↑cMyc↑G₃₂₂). The resulting plasmids, namely, pESC-URAA/P2(H₂₅₃↑Flag↑G₂₅₄/A₃₂₁↑Flag↑G₃₂₂), pESC-URA/VP2(H₂₅₃↑Flag↑G₂₅₄/A₃₂₁↑ cMyc↑G₃₂₂), pESC-URA/VP2 (H₂₅₃↑cMyc↑G₂₅₄/ A₃₂₁↑Flag↑G₃₂₂), and pESC-URA/VP2(H₂₅₃↑ cMyc↑G₂₅₄/A₃₂₁↑cMyc↑G₃₂₂), were used to transform *S.cerevisiae* Y449 strain to evaluate VP2 expression and chimeric VP2 VLP formation capacity by quantitative VLP-ELISA. As shown in Table 8, VP2 452 proteins with two P loop region insertions are capable of forming VLP. In general it was observed that the second insertion reduced VLP formation efficiency slightly but VLP were formed in all cases with efficiencies equal or above 20%, and the presence of VLP was confirmed by EM analysis.

**Table 8: VLP production capacity of VP2 constructs with multiple insertions.**

| (Core peptides are shown in bold and underlined; %VLP: VLP Formation Efficiency). | | |
|---|---|---|
| INSERTION POSITION (INSERTED PEPTIDE SEQUENCE) | INSERTION POSITION (INSERTED PEPTIDE SEQUENCE) | %VLP |
| H₂₅₃↑Flag↑G₂₅₄ (TSDYKDDDDKGSGGSSDYKDDDDKTS) | none | 100 |
| | A₃₂₁↑Flag↑G₃₂₂ (TSDYKDDDDKGSGGSSDYKDDDDKTS) | 66 |
| | A₃₂₁↑cMyc↑G₃₂₂ (TSEQKLISEEDLTS) | 45 |
| H₂₅₃↑cMyc↑G₂₅₄ (TSEQKLISEEDLTS) | none | 84 |
| | A₃₂₁↑Flag↑G₃₂₂ (TSDYKDDDDKGSGGSSDYKDDDDKTS) | 33 |
| | A₃₂₁↑cMyc↑G₃₂₂ (TSEQKLISEEDLTS) | 82 |
| none | A₃₂₁↑Flag↑G₃₂₂ (TSDYKDDDDKGSGGSSDYKDDDDKTS) | 62 |
| | A₃₂₁↑cMyc↑G₃₂₂ (TSEQKLISEEDLTS) | 67 |

### Example 9: Chimeric VP2 VLP resulting from chimeric VP2 fusion proteins incorporating the insertion or substitution with a peptide of interest in addition to a terminally fused peptide of interest.

To test the possibility of combining VP2 insertions or substitutions with terminal fusions, a series of VP2 insertion or substitution constructs with N- or C- terminal peptide fusions were prepared as shown in Figure 5. For the construction of a C-terminal fusion site DNA constructs incorporating the insertion of a DNA of interest, Flag or cMyc, within VP2 at the H₂₅₃↑G₂₅₄ or A₃₂₁↑G₃₂₂ site and DNA constructs encoding for a 4K substitution mutant, containing four lysine residues in P loop regions, were genetically engineered to contain unique *Not*I and *Hind*III restriction sites at the C-terminal end of the VP2 gene [e.g.: pESC-URA/VP2(H₂₅₃↑cMyc↑G₂₅₄)-*Not Hind;* and pESC-URA/VP2(Q₂₂₁K-H₂₅₃K-G₂₈₅K-H₃₂₀K)-*Not Hind*]. Double digest of the VP2 insertion or substitution vectors with *Not*I *- Hind*III and re-ligation to incorporate a *Not*I *- Hind*III linear fragment encoding for cMyc or Flag, resulted in the C-terminal fusion of the second, or same, DNA of interest or in the combination of the C-terminal fusion and 4K substitutions [e.g.: pESC-URA/VP2(H₂₅₃↑Flag↑G₂₅₄)-Flag; or pESC-URA/VP2(Q₂₂₁K-H₂₅₃K-G₂₈₅K-H₃₂₀K)-cMyc].

For the construction of N- terminal fusions DNA constructs incorporating the insertion of a DNA of interest, Flag (SEQ. ID. NO: 2) or cMyc (SEQ. ID. NO: 3), within VP2 at the H₂₅₃↑G₂₅₄ or A₃₂₁↑G₃₂₂, H₂₅₃↑G₂₅₄ site and DNA constructs encoding for the 4K substitution mutant were digested with *EcoR*I*,* positioned immediately upstream of the start-codon, and ligated to a Flag encoding DNA fragment with *EcoR*I sticky ends. Transformation of the ligation product into supercompetent *E.coli* led to the isolation plasmids encoding mutants with N- terminal Flag fusions in combination with VP2 insertions or substitutions. [e.g.: pESC-URA/Flag-VP2(H₂₅₃↑Flag↑G₂₅₄); or pESC-URA/Flag-VP2(Q₂₂₁K-H₂₅₃K-G₂₈₅K-H₃₂₀K)]. Transformation of all constructs into *S.cerevisiae* Y449 and isolation of transformed yeast colonies was followed by evaluation of VLP production efficiency, as shown in Table 11. In general fusion of a peptide of interest to the C-terminus reduced VLP formation efficiency but VLP were formed in all cases with efficiencies equal or above 20%, and the presence of VLP was confirmed by electron microscopy (EM).

**Table 9. VLP production capacity of VP2 constructs incorporating the insertion of a peptide of interest and a C- or N- terminal fusion of a second peptide of interest.**

| (%VLP: VLP Formation Efficiency; BG: Background expression; NT: Not Tested) | | |
|---|---|---|
| INSERTION POSITION (INSERTED PEPTIDE SEQUENCE) | TERMINAL FUSION POSITION (INSERTED PEPTIDE SEQUENCE) | %VLP |
| H₂₅₃↑Flag↑G₂₅₄ (TSDYKDDDDKGSGGSSDYKDDDDKSTS ) | None | 100 |
| | R₄₅₂↑Flag(SSGDYKDDDDK) | 23 |
| | R₄₅₂↑cMyc(SSGDYKDDDDK) | 30 |
| | Flag↑M₁(MDYKDDDDKGNSEF) | 45 |
| A₃₂₁↑Flag↑G₃₂₂ (TSDYKDDDDKGSGGSSDYKDDDDKSTS ) | None | 62 |
| | R₄₅₂↑Flag(SSGDYKDDDDK) | 67 |
| | R₄₅₂↑cMyc(SSGDYKDDDDK) | NT |
| | Flag↑M₁(MDYKDDDDKGNSEF) | 34 |
| H₂₅₃↑cMyc↑G₂₅₄ (TSEQKLISEEDLSTS) | None | 84 |
| | R₄₅₂↑Flag(SSGDYKDDDDK) | 55 |
| | R₄₅₂↑cMyc(SSGDYKDDDDK) | 30 |
| | Flag↑M₁↑(MDYKDDDDKGNSEF) | 78 |
| A₃₂₁↑cMyc↑G₃₂₂ (TSEQKLISEEDLTSTS) | None | 67 |
| | R₄₅₂↑Flag(SSGDYKDDDDK) | 85 |
| | R₄₅₂↑cMyc(SSGDYKDDDDK) | NT |
| | Flag↑M₁(MDYKDDDDKGNSEF) | 56 |
| Q₂₂₁K-H₂₅₃K-G₂₈₅K-H₃₂₀K (4K) | None | 45 |
| | R₄₅₂↑Flag(SSGDYKDDDDK) | 34 |
| | R₄₅₂↑cMyc(SSGDYKDDDDK) | 40 |
| | Flag↑M₁(MDYKDDDDKGNSEF) | 55 |

### Example 10: Production in baculovirus of T=13 VLP incorporating VP2 insertions and VP3 terminal fusions by expression chimeric pVP2-VP4-VP3 poly-protein genes.

For the generation of IBDV T=13 VLP incorporating insertions of peptides of interest within VP2, plasmids were constructed for the expression of the IBDV pVP2-VP4-VP3 polyprotein. More specifically, baculovirus expression plasmids, namely pFastBacDual (pFBD) from Invitrogen™, and synthetic pVP2 (SEQ. ID. NO: 21), VP4 (SEQ. ID. NO:23) and VP3 (SEQ. ID. NO: 25) genes of IBDV Soroa strain cloned into pUC57 (NCIB No. AAD30136) as shown in Figures 6, 7 and 8, were used for the construction pFBD/pVP2-VP4-VP3 polyproteins plasmids [i.e.: pFBD/pVP2-VP4-VP3-pp]. Construction of plasmids is outlined in Figure 9 and as follows:
In a first step, the gene encoding for the full length 512 amino acid pVP2 (SEQ. ID. NO: 21) was the cloned into pFBD plasmid downstream of the PH promoter. Plasmid pUC57-pVP2 was digested with restriction enzymes *Bg*/II and *Hind*III and the DNA fragment was cloned into *Bam*HI and *Hind*III digested pFBD plasmid generating pFBD/pVP2-pp.

In a second step the VP3 fusion proteins genes were cloned into pFBD/VP2 downstream of the pVP2 gene. DNA fragments encoding for VP3-Flag and VP3-cMyc were obtained by PCR using the synthetic VP3 gene (pUC57-VP3) shown in Figure 8 as template and oligonucleotide primers BV VP3 5' *Hind*III (SEQ. ID. NO: 10) and BV VP3-Flag 3'*Hind*III (SEQ. ID. NO: 11), BV VP3-cMyc 3'*Hind*III (SEQ. ID. NO: 12) respectively as shown in Figure 9. Purified DNA fragments were digested with restriction enzymes *Hind*III and cloned into *Hind*III digested pFBD/pVP2-pp generating pFBD/pVP2-VP3-Flag-pp and pFBD/pVP2-VP3-cMyc-pp.

In a third step, DNA fragments encoding for the VP2 insertions were cloned into pFBD/pVP2-VP3(X)-pp plasmids. For this purpose loop insertion plasmids [e.g.: pFBD/VP2(H₂₅₃↑Flag↑G₂₅₄); pFBD/VP2(H₂₅₃↑cMyc↑G₂₅₄); or pFBD/VP2(Q₂₂₁K-H₂₅₃K-G₂₈₅K-H₃₂₀K)] were digested with the restriction enzyme *Rsr*II and *Nco*I, which cut within the VP2 gene at amino acid positions G₂₄-H₃₃₈ and the purified VP2 gene fragment was cloned into *Rsr*II and *Nco*I digested pFBD/pVP2-VP3-Flag-pp and pFBD/pVP2-VP3-cMyc-pp plasmids, generating plasmids pFBD/pVP2(H₂₅₃↑Flag or Myc↑G₂₅₄)-VP3-Flag-pp, pFBD/pVP2(H₂₅₃↑Flag or Myc↑G₂₅₄)-VP3-cMyc-pp, pFBD/pVP2(A₃₂₁↑Flag or Myc↑G₃₂₂)-VP3-Flag-pp, pFBD/pVP2(A₃₂₁↑Flag or Myc↑G₃₂₂)-VP3-cMyc-pp, pFBD/pVP2(Q₂₂₁K-H₂₅₃K-G₂₈₅K-H₃₂₀K)-VP3-Flag-pp and pFBD/pVP2(Q₂₂₁K-H₂₅₃K-G₂₈₅K-H₃₂₀-K)-VP3-cMyc-pp, as shown in Figure 9.

In a final step of the construction the VP4 gene was inserted between the pVP2 and VP3 genes to create the pVP2-VP4-VP3 open reading frame. To carry this out, plasmid pUC57-VP4 was digested with restriction enzymes *Pvu*II and *Bsg*I and the pVP4 DNA fragment was cloned into *Pvu*II and *Bsg*I digested plasmids pFBD/pVP2(X)-VP3-X (e.g. pFBD/pVP2(H₂₅₃↑Flag or Myc↑G₂₅₄)-VP3-Flag-pp and pFBD/pVP2(Q₂₂₁K-H₂₅₃K-G₂₈₅K-H₃₂₀-K)-VP3-cMyc-pp)

The resulting plasmids [e.g.: pFBD/pVP2(H₂₅₃↑Flag or Myc↑G₂₅₄)-VP4-VP3-Flag-pp and pFBD/pVP2(Q₂₂₁K-H₂₅₃K-G₂₈₅K-H₃₂₀-K)-VP4-VP3-cMyc-pp] were introduced in recombinant Baculoviruses (rBV) which in the course of their replicating cycle expressed the pVP2(X)-VP4-VP3-X polyproteins. VLP production in rBV infected insect cells and T=13 VLP purification was carried out following standard procedures.

Briefly, cultures of H5 insect cells (Invitrogen™) were infected with rBV at a multiplicity of infection of 5 pfu/cell. At 30 h post infection, cells were harvested, lysed, and VLP purified by sucrose gradient centrifugation. T13 VLP formation capacity for all constructs was evaluated by VLP-ELISA and EM of purified VLP samples. As shown in Table 10, all tested constructs resulted in T=13 VLP, indicating that chimeric VP2 fusion proteins containing insertions are indeed compatible with T=13 VLP formation and furthermore that T=13 VLP can be made to incorporate the combination of peptides of interests inserted in VP2 and fused to VP3.

**Table 10: VLP production capacity of T=13 VLP incorporating VP2 insertions and VP3 terminal fusions and produced by expression pVP2-VP4-VP3 poly-protein gene in baculovirus.**

| (* % VLP formation efficiency compared to pVP2-HisVP3 generated by co-expression pVP2 and VP3). | | | |
|---|---|---|---|
| INSERTION POSITION (INSERTED PEPTIDE SEQUENCE) | VP3 fusion | POSITION/PEPTIDE SEQUENCES | %VLP * |
| H₂₅₃↑Flag↑G₂₅₄ (TSDYKDDDDKGSGGSSDYKDDDDKTS) | VP3-Flag | E₂₅₉/SSGDYKDDDDK | 80 |
| | VP3-cMyc | E₂₅₉/EQKLISEEDL | 92 |
| H₂₅₃↑cMyc↑G₂₅₄ (TSEQKLISEEDLTS) | VP3-Flag | E₂₅₉/SSGDYKDDDDK | 77 |
| | VP3-cMyc | E₂₅₉/EQKLISEEDL | 55 |
| A₃₂₁↑Flag↑G₃₂₂ (TSDYKDDDDKGSGGSSDYKDDDDKTS) | VP3-Flag | E₂₅₉/SSGDYKDDDDK | 44 |
| | VP3-cMyc | E₂₅₉/EQKLISEEDL | 77 |
| A₃₂₁↑cMyc↑G₃₂₂ | VP3-Flag | E₂₅₉/SSGDYKDDDDK | 82 |
| (TSEQKLISEEDLTS) | VP3-cMyc | E₂₅₉/EQKLISEEDL | 76 |
| Q₂₂₁K-H₂₅₃K-G₂₈₅K-H₃₂₀K (4K) | VP3-Flag | E₂₅₉/SSGDYKDDDDK | 66 |
| | VP3-cMyc | E₂₅₉/EQKLISEEDL | 45 |

### Example 11: Production in yeast S.cerevisiae of T=13 VLP incorporating a VP2 insertion and VP3 terminally fused to N-and C-terminus.

For the generation of T=13 VLP incorporating insertions of peptides of interest within VP2, plasmids were constructed for the co-expression of VP2 and VP3 from separate genes. Single expression plasmids were used, namely pESC-URA from Stratagene™, that permitted expression in yeast. Synthetic genes for the pVP2-512 (SEQ. ID. NO: 21) and VP3 (SEQ. ID. NO: 25) of IBDV Soroa strain were cloned into pUC57 (NCIB No. AAD30136) as shown in Figures 6 and 8, and were used for the plasmid constructions. Construction of plasmids is outlined in Figure 10.

In a first step, the construction of pESC-URA/pVP2-VP3 plasmids VP3 fusion proteins genes were cloned into pESC-URA downstream of the GAL10 promoter (MSC1). DNA fragments encoding for His-VP3, His-VP3-Flag and His-VP3-cMyc were obtained by PCR using the synthetic VP3 gene (pUC57-VP3) shown in Figure 8 as template and oligonucleotide primers Y His-VP3 5'(SEQ. ID. NO: 13) and Y VP3 3'(SEQ. ID. NO: 14), Y VP3-Flag 3'(SEQ. ID. NO: 15), Y VP3-cMyc 3 (SEQ. ID. NO: 16), respectively as shown in Figure 1. Purified DNA fragments were digested with restriction enzymes *Not*I and Sacl and cloned into *Not*I and *Sac*I digested pESC-URA generating the following pESC-URA/ His-VP3(X) plasmids, pESC-URA/ His-VP3, pESC-URA/ His-VP3-Flag and pESC-URA/ His-VP3-cMyc.

In a second step, the gene encoding for the full length 512 amino acid pVP2 was the cloned into pESC-URA/His-VP3-X plasmids, were X is example DNA of interest, namely cMyc (SEQ. ID. NO: 3) or Flag (SEQ. ID. NO: 2), downstream of the GAL1 promoter (MCS2). Plasmid pUC57-pVP2 was digested with restriction enzymes *Bgl*II and *Hind*III and the DNA fragment was cloned into *Bam*HI and *Hind*III digested pESC-URA/His-VP3-X plasmids, generating the following pESC-URA/pVP2-VP3-X plasmids, pESC-URA/pVP2-His-VP3, pESC-URA/pVP2-His-VP3-Flag, and pESC-URA/pVP2-His-VP3-cMyc.

In a third step, DNA fragments encoding for the VP2 insertions were cloned into pESC-URA/pVP2-His-VP3-X plasmids. For this purpose loop insertion plasmids [e.g.: pESC-URA/VP2(H₂₅₃↑Flag↑G₂₅₄), or pESC-URA/VP2(Q₂₂₁K -H₂₅₃K-G₂₈₅K-H₃₂₀K)] were digested with the restriction enzymes *Rsr*II and *Msc*I, which cut within the VP2 gene at amino acid positions G₂₄-W₄₁₄ and the purified VP2 gene fragment was cloned into *Rsr*II and *Msc*I digested pESC-URA/pVP2-His-VP3-X plasmids.

The resulting plasmids, namely pESC-URA/pVP2(H₂₅₃↑Flag or cMyc↑G₂₅₄)-His-VP3, pESC-URA/pVP2(A₃₂₁↑Flag or cMyc↑G₃₂₂)-His-VP3, pESC-URA/pVP2(H₂₅₃↑Flag or cMyc↑G₂₅₄)-His-VP3-Flag, pESC-URA/pVP2(H₂₅₃↑Flag or cMyc↑G₂₅₄)-His-VP3-cMyc, pESC-URA/pVP2(A₃₂₁↑Flag or cMyc↑G₃₂₂)-His-VP3-Flag, pESC-URA/pVP2(A₃₂₁↑Flag or cMyc↑G₃₂₂)-His-VP3-cMyc, pESC-URA/VP2(Q₂₂K-H₂₅₃K-G₂₈₅K-H₃₂₀K)-His-VP3, pESC-URA/VP2(Q₂₂₁K-H₂₅₃K-G₂₈₅K-H₃₂₀K)-His-VP3-Flag and pESC-URA/VP2(Q₂₂₁K-H₂₅₃K-G₂₈₅K-H₃₂₀K)-His-VP3-cMyc, were used to transform *S.cerevisiae* Y449 to evaluate for VP2/VP3 expression and VLP formation capacity by quantitative VLP-ELISA using total yeast cell extracts. The formation of T=13 was confirmed by EM of purified VLP samples and compared to VLP formation of a pVP2-His-VP3 construct lacking VP2 insertions. As shown in Table 11 all tested constructs resulted in the formation of T=13 VLP with varying degrees of efficiency above 20% indicating that chimeric VP2 fusion proteins containing insertions are indeed compatible with T=13 VLP formation in yeast, and furthermore that T=13 VLP can be made to incorporate chimeric VP2 fusion proteins and chimeric VP3.

**Table 11. VLP production capacity of T=13 VLP incorporating VP2 insertions and VP3 terminal fusions generated by expression of pVP2 and VP3 in yeast.**

| (* % VLP formation efficiency compared to pVP2-HisVP3 generated by co-expression pVP2 and VP3). | | | |
|---|---|---|---|
| INSERTION POSITION (INSERTED PEPTIDE SEQUENCE) | VP3fusion | POSITION / PEPTIDE SEQUENECES | %VLP * |
| H₂₅₃↑Flag↑G₂₅₄ (TSDYKDDDDKGSGGSSDYKD DDDKSTS) | His-VP3 | His-VP3:M₁/HHHHHHSSG | 44 |
| | His-VP3-Flag | M₁/HHHHHHSSG,E₂₅₉/SSGDYKDDDDK | 23 |
| | His-VP3-cMyc | M₁/HHHHHHSSG,E₂₅₉/EQKLISEEDL | 12 |
| H₂₅₃↑cMyc↑G₂₅₄ (TSEQKLISEEDLSTS) | His-VP3 | His-VP3:M₁/HHHHHHSSG | 66 |
| | His-VP3-Flag | M₁/HHHHHHSSG,E₂₅₉/SSGDYKDDDDK | 62 |
| | His-VP3-cMyc | M₁/HHHHHHSSG,E₂₅₉/EQKLISEEDL | 44 |
| A₃₂₁↑Flag↑G₃₂₂ (TSDYKDDDDKGSGGSSDYKD DDDKSTS) | His-VP3 | His-VP3:M₁/HHHHHHSSG | 53 |
| | His-VP3-Flag | M₁/HHHHHHSSG,E₂₅₉/SSGDYKDDDDK | 42 |
| | His-VP3-cMyc | M₁/HHHHHHSSG,E₂₅₉/EQKLISEEDL | 66 |
| A₃₂₁↑cMyc↑G₃₂₂ (TSEQKLISEEDLSTS) | His-VP3 | His-VP3:M₁/HHHHHHSSG | 22 |
| | His-VP3-Flag | M₁/HHHHHHSSG,E₂₅₉/SSGDYKDDDDK | 12 |
| | His-VP3-cMyc | M₁/HHHHHHSSG,E₂₅₉/EQKLISEEDL | 59 |
| Q₂₂₁K-H₂₅₃K-G₂₈₅K-H₃₂₀K (4K) | His-VP3 | His-VP3:M₁/HHHHHHSSG | 55 |
| | His-VP3-Flag | M₁/HHHHHHSSG,E₂₅₉/SSGDYKDDDDK | 33 |
| | His-VP3-cMyc | M₁/HHHHHHSSG,E₂₅₉/EQKLISEEDL | 56 |

### Example 12: Expression of chimeric VP2 fusion proteins and VP3 in baculovirus for production of T=13 VLP incorporating VP2 insertions and VP3 terminal fusions.

For the generation of IBDV T=13 VLP incorporating insertions of peptides of interest within VP2, plasmids were constructed for the co-expression of IBDV VP2 and VP3 from separate genes. Single expression plasmids were used, namely pFastBacDual (pFBD) from Invitrogen™, which permitted expression in insect cells. Synthetic genes for the pVP2 512 (SEQ. ID. NO: 21) and VP3 (SEQ. ID. NO: 25) of IBDV Soroa strain cloned into pUC57 (NCIB No. AAD30136) as shown in Figures 6 and 8, were used for the plasmid constructions (see Figure 11) as follows:
In a first step in the construction of pFBD/pVP2-VP3 plasmids, VP3 fusion proteins genes were cloned into pFBD downstream of the p10 promoter. DNA fragments encoding for His-VP3, His-VP3-Flag and His-VP3-cMyc were obtained by PCR using the synthetic VP3 gene (pUC57-VP3) shown in Figure 6 as template and oligonucleotide primers BV His-VP3 5'(SEQ. ID. NO: 17) and BV VP3 3'(SEQ. ID. NO: 18), BV VP3-Flag 3'(SEQ. ID. NO: 19), BV VP3-cMyc 3'(SEQ. ID. NO: 20), respectively as shown in Figure 2.

Purified DNA fragments were digested with restriction enzymes *Sma*I and *Kpn*I and cloned into *Sma*I and *Kpn*I digested pFBD generating pFBD/His-VP3-X plasmids, were X is DNA of interest, namely Flag (SEQ. ID. NO: 2) of cMyc(SEQ. ID. NO: 3).

In a second step, the gene encoding for the full length 512 amino acid VP2 was cloned into pFBD/ His-VP3-X plasmids downstream of the PH promoter. Plasmid pUC57-pVP2 was digested with restriction enzymes *Bgl*II and *Hind*III and the DNA fragment was cloned into *Bam*HI and *Hind*III digested pFBD/VP3-X plasmids generating the following pFBD/pVP2- His-VP3-X plasmids, pFBD/pVP2-His-VP3, pFBD/pVP2- His-VP3-Flag, and pFBD/pVP2- His-VP3-cMyc.

In a third step, DNA fragments encoding for the VP2 insertions were cloned into pFBD/pVP2-VP3-X plasmids. For this purpose loop insertion plasmids [e.g.: pESC-URA/VP2(H₂₅₃↑Flag↑G₂₅₄); pESC-URA/VP2(H₂₅₃↑cMyc↑G₂₅₄ or pESC-URA/VP2/Q₂₂₁K-H₂₅₃K-G₂₈₅K-H₃₂₀K] were digested with the restriction enzyme *Rsr*II and *Nco*I, which cut within the VP2 gene at amino acid positions G₂₄-H₃₃₈ and the purified VP2 gene fragment was cloned into *Rsr*II and *Nco*I digested pFBD/pVP2-His-VP3-X plasmids.

The resulting plasmids, shown in Figure 11, namely pFBD/pVP2(H₂₅₃↑Flag or cMyc↑G₂₅₄)-His-VP3, pFBD/pVP2(H₂₅₃↑Flag or cMyc↑G₂₅₄)-His-VP3-Flag, pFBD/pVP2(H₂₅₃↑Flag or Myc↑G₂₅₄)-His-VP3-cMyc, pFBD/pVP2(Q₂₂₁K-H₂₅₃K-G₂₈₅K-H₃₂₀K)-His-VP3, pFBD/pVP2(Q₂₂₁K-H₂₅₃K-G₂₈₅K-H₃₂₀K)-VP3-Flag and pFBD/pVP2(Q₂₂₁K-H₂₅₃K-G₂₈₅K-H₃₂₀K)-His-VP3-cMyc, were introduced in recombinant Baculoviruses (rBV) which in the course of their replicating cycle expressed both proteins, VP2 fusions and VP3 fusions, simultaneously. The same procedure was carried out with Flag and cMyc VP2 insertions at location A₃₂₁↑G₃₂₂ as shown in Figure 11. VLP production in rBV infected insect cells and T=13 VLP purification was carried out following standard procedures. Briefly, cultures of H5 insect cells (Invitrogen™) were infected with rBV at a multiplicity of infection of 5 pfu/cell. At 30 hr post infection, cells were harvested, lysed, and VLP purified by sucrose gradient centrifugation. T13 VLP formation capacity for all constructs was evaluated by ELISA and TEM of purified VLP samples and compared to VLP formation of a pVP2-His-VP3 construct lacking VP2 insertions (see Figure 12). As shown in Table 12, all tested constructs resulted in T=13 VLP with formation efficiencies above 20%, indicating that VP2 insertions are indeed compatible with T=13 VLP formation in Baculovirus infected cells and furthermore that T=13 VLP can be made to incorporate the combination of peptides of interests inserted in V=P2 and fused to VP3.

**Table 12: VLP production capacity of T=13 VLP incorporating VP2 insertions and VP3 terminal fusions generated by co-expression of pVP2 and VP3.**

| (* % VLP formation efficiency compared to pVP2-HisVP3 generated by co-expression pVP2 and VP3). | | | |
|---|---|---|---|
| INSERTION POSITION (INSERTED PEPTIDE SEQUENCE) | VP3 fusion | POSITION / PEPTIDE SEQUENECES | % VLP * |
| H₂₅₃↑Flag↑G₂₅₄ (TSDYKDDDDKGSGGSSDYKDDDD KSTS) | His-VP3 | His-VP3:M₁/HHHHHHSSG | 66 |
| | His-VP3-Flag | M₁/HHHHHHSSG,E₂₅₉/SSGDYKDDDDK | 45 |
| | His-VP3-cMyc | M₁/HHHHHHSSG,E₂₅₉/EQKLISEEDL | 35 |
| H₂₅₃↑cMyc↑G₂₅₄ (TSEQKLISEEDLSTS) | His-VP3 | His-VP3:M₁/HHHHHHSSG | 78 |
| | His-VP3-Flag | M₁/HHHHHHSSG,E₂₅₉/SSGDYKDDDDK | 55 |
| | His-VP3-cMyc | M₁/HHHHHHSSG,E₂₅₉/EQKLISEEDL | 34 |
| A₃₂₁↑Flag↑G₃₂₂ (TSDYKDDDDKGSGGSSDYKDDDD KSTS) | His-VP3 | His-VP3:M₁/HHHHHHSSG | 78 |
| | His-VP3-Flag | M₁/HHHHHHSSG,E₂₅₉/SSGDYKDDDDK | 22 |
| | His-VP3-cMyc | M₁/HHHHHHSSG,E₂₅₉/EQKLISEEDL | 88 |
| A₃₂₁↑cMyc↑G₃₂₂ (TSEQKLISEEDLSTS) | His-VP3 | His-VP3:M₁/HHHHHHSSG | 33 |
| | His-VP3-Flag | M₁/HHHHHHSSG,E₂₅₉/SSGDYKDDDDK | 56 |
| | His-VP3-cMyc | M₁/HHHHHHSSG,E₂₅₉/EQKLISEEDL | 88 |
| Q₂₂₁K-H₂₅₃K-G₂₈₅K-H₃₂₀K (4K) | His-VP3 | His-VP3:M₁/HHHHHHSSG | 55 |
| | His-VP3-Flag | M₁/HHHHHHSSG,E₂₅₉/SSGDYKDDDDK | 33 |
| | His-VP3-cMyc | M₁/HHHHHHSSG,E₂₅₉/EQKLISEEDL | 56 |

### Example 13: Array Screen for insertions against a library incorporating previously identified VP2 insertion vectors.

To limit the number of false positive hits during the screens, a second generation of VP2 loop insertion vectors was prepared to contain a multiple cloning site (MCS) for the efficient directional cloning of a DNA of interest. For this purpose vectors containing a *Spe*I site at each of the possible VP2 insertion positions were digested with *Spe*I and re-ligated to incorporate a MCS insert which allowed the directional cloning of DNA of interest using restriction sites *Not*I and *Spe*I (See Figure 13). The MCS insert contained several in-frame stop codons to ensure that re-ligation of the MSC containing vectors encoded for a deleted VP2 protein unable to assemble in the form of VLP.

For the performed array test screen a selection of 7 VP2 MCS insertion vectors were plated in the form of an array, one vector per well, as triplicates in 96-well plates. The selection of vectors included constructs with insertion sites at VP2 P loop regions or outside said VP2 P loop regions. Vectors were simultaneously digested with *Not*I and *Spe*I for 2 hr in appropriate buffer conditions. Following heat inactivation at 65°C for 30 minutes, a purified DNA fragment of the peptide of interest, namely Flag (SEQ. ID. NO: 2) and cMyc (SEQ. ID. NO: 3), with *Not*I and *Spe*I sticky ends was added and ligation performed with T4 ligase at 16 °C for 18 hr. To each of the wells 200 □l of supercompetent *S.cerevisiae* Y449 were added for transformation followed after 30 minutes by addition of 750 ul of selective culture medium. After cultivation at 30°C for 16 hr, cells were lysed by 3 freeze-thaw cycles and VLP formation efficiency was tested by quantitative VLP-ELISA. VLP expression efficiency for each of the clones varied for the different vectors according to the site of insertion as shown in Table 13.

The VLP formation efficiency for each construct was calculated as an average value of the triplicate wells. The data obtained for the insertion vectors H₂₅₃↑G₂₅₄, F₂₄₉ ↑R₂₅₀, T₂₈₆↑D₂₈₇, D₃₂₃↑Q₃₂₄ and G₁₈₈↑L₁₈₉ agreed well with VLP formation data previously obtained for these vectors (see table 1), indicating that the array screen can accurately predict accurately the optimal insertion site for peptide of interest and that array screens are indeed a useful tool for the identification of preffered insertion site for a particular peptide of interest.

**Table 13: VLP expression following insertion of a peptide of interest into VP2 insertion vectors arranged in an array format.**

| (%VLP: VLP Formation Efficiency) | | | | |
|---|---|---|---|---|
| WELL | POSITION | LOCATION / DESCRIPTION | % VLP | |
| | | | Flag peptide | cMyc peptide |
| 1 | H₂₅₃↑G₂₅₄ | Surface P loop DE | 88 | 85 |
| 2 | F₂₄₉↑R₂₅₀ | Surface P loop DE | BG | 25 |
| 3 | T₂₈₆↑D₂₈₇ | Surface P loop FG | 70 | 45 |
| 4 | D₃₂₃↑Q₃₂₄ | Surface P loop HI | 55 | 85 |
| 5 | Q₇₆↑N₇₇ | Outside P loop | 77 | 54 |
| 6 | S₁₁₁↑T₁₁₂ | Outside P loop | 55 | 67 |
| 7 | G₁₈₈↑L₁₈₉ | OutsideP loop | 34 | 45 |

### Example 14: Pool screen for Flag insertions of against a library incorporating previously identified VP2 insertion vectors.

For the pool screen, an equimolar selection of plasmid DNA of 7 previously selected VP2 insertion vectors containing a MCS, including a *Not*I/*Spe*I cloning site at the desired insertion site, were mixed. The selection of vectors included P loop and outside P loop region cloning sites as shown in Table 14. Pooled vectors were digested with *Not*I and *Spe*I for 2 hr in appropriate buffer conditions, incubated at 65°C for 30 minutes and purified by gel electrophoresis. A linear fragment, with *Not*I and *Spe*I sticky ends, of the DNA of interest encoding for Flag (SEQ. ID. NO: 2) as an example peptide of interest, was mixed with the pool of digested vectors and ligated with T4 ligase at 16 °C for 18 hr. Transformation of electrocompetent *E.coli* with 1 ul of ligation mixture resulted in a VP2 insertion library for the peptide of interest containing insertion at the 10 different pre-selected locations. Pooled plasmid DNA was purified And 10 µg transformed into *S.cerevisiae* Y449 yeast and plated on YNB/CSM-URA+2% glucose. Isolated yeast clones were transferred to galactose-containing plates and resulting individual colonies were transferred to a PVDF membrane to analyze for VLP production by colony immunoblot against VP2 VLP specific antibodies. A selection of 50 positive clones were picked and grown individually in selective liquid medium (YNB/CSM-URA+2% galactose) and quantitative VLP-ELISA was carried out to confirm VLP production of positive clones. Twenty colonies with VLP formation efficiencies above 50 % were sequenced to identify the site of insertion within VP2. The confirmed positive clones had insertions at sites previously identified as preferred for insertions [e.g.: H₂₅₃↑G₂₅₄, D₃₂₃↑Q₃₂₄, Q₇₆↑N₇₇)] shown in Table 14. On the other hand, no clones with insertions at a less preferred insertion site [e.g.: F₂₄₉↑R₂₅₀] were identified in the pool screen, indicating that the array screen could predict accurately the optimal insertion site for a peptide of interest and that pool screens are indeed a useful tool for the identification of preferred insertion sites for a particular peptide of interest.

**Table 14: Preferred VP2 VLP insertion sites for Flag resulting from a screen against a pool of 7 previously identified VP2 insertion vectors.**

| POSITION | LOCATION DESCRIPTION | RESULT OF POOL SCREEN / Number of identified clones with VLP formation efficiency >50% of VP2 452 control |
|---|---|---|
| H₂₅₃↑G₂₅₄ | P loop DE | 6 |
| F₂₄₉↑R₂₅₀ | P loop DE | 0 |
| T₂₈₆↑D₂₈₇ | P loop FG | 2 |
| D₃₂₃↑Q₃₂₄ | P loop HI | 8 |
| Q₇₆↑N₇₇ | Outside P loop | 4 |
| S₁₁₁↑T₁₁₂ | Outside P loop | 3 |
| G₁₈₈↑L₁₈₉ | OutsideP loop | 1 |

### Example 15: Random transposon-based peptide insertion screen.

A VP2 452 library containing random insertions of Mu transposon along the entire VP2 452 was digested with *Not*I at the unique site provided by the Mu transposon. The linearised plasmid pool was re-ligated to incorporate three DNA adapters with *Not*I sticky ends encoding for the Flag (SEQ. ID. NO: 2) or cMyc (SEQ. ID. NO: 3) peptide in each of the three possible reading frames. 0.5-1 µl of the ligation mixture was used to transform *Transformax EC100 Electrocompetent E.coli* to obtain a random VP2 Flag insertion library in *E.coli.* The library was expanded and pooled plasmid DNA purified and 10 µg used to transform *S.cerevisiae* Y449 yeast prior to plating onto YNB/CSM-URA+2% glucose plates. 10,000 yeast clones were obtained and were transferred to galactose-containing selection plates. Colonies grown in the presence of galactose were transferred to a PVDF membrane to analyze for VP2 expression and VLP formation by colony immunoblot. A selection of 180 positive clones were growth individually in selective liquid medium (YNB/CSM-URA+2% galactose) and quantitative VLP-ELISA was carried out to confirm VLP production of positive clones. 16 samples of the selected positive clones of the Flag and cMyc screen showed VLP formation efficiencies above 50 % of the VP2 452 control and the chimeric VP2 gene of these clones was sequenced to identify the site of insertion. Table 15 shows VLP production capacity of Flag peptide VP2 insertions resulting from a random Transposon based peptide insertion screen.

As shown in Table 15, some of the confirmed positive clones contained the insertion of the peptide of interest, Flag (SEQ. ID. NO: 2), at previously identified preferred sites such as M₁↑T₂, I₁₈₄↑P₁₈₅, G₁₈₈↑L₁₈₉, L₄₃₆↑K₄₃₇ while others showed insertions at previously unidentified locations outside the P loop regions. Two clones at insertion positions I₁₈₄↑P₁₈₅, L₃₇₉↑A₃₈₀ showed the insertion of two copies indicating that at these could be preferred insertions site for large inserts. Three independent Flag insertion clone were found at two previously identified insertion site (I₁₈₄↑P₁₈₅, L₄₃₆↑K₄₃₇) suggesting that these sites constitute a preferred insertion site outside the P loop region.

**Table 15: VLP production capacity of Flag peptide VP2 insertions resulting from a random Transposon based peptide insertion screen.**

| (Inserted peptides of interest are shown underlined with the reminder amino acids resulting from the inserted Mu transposon; %VLP: VLP formation efficiency). | | |
|---|---|---|
| INSERTION POSITION | INSERTED SEQUENCE | % VLP |
| M₁↑T₂ | MMRP**DYKDDDDKGSGGSSDYKDDDDKS**RPHMT | 77 |
| Q₉↑Q₁₀ | QCGP**DYKDDDDKGSGGSSDYKDDDDKS**GRTQQ | 79 |
| I₁₈₄↑P₁₈₅ | | 87 |
| I₁₈₄↑P₁₈₅ | ICGP**DYKDDDDKGSGGSSDYKDDDDKS**GRTIP | 63 |
| I₁₈₄↑P₁₈₅ | ICGP**DYKDDDDKGSGGSSDYKDDDDKS**GRTIP | 54 |
| G₁₈₈↑L₁₈₉ | GCGP**DYKDDDDKGSGGSSDYKDDDDKS**GRIGL | 85 |
| A₂₁₁↑D₂₁₂ | AAA**DYKDDDDKGSGGSSDYKDDDDKS**PAAAAD | 59 |
| D₂₈₇↑N₂₈₈ | DAA**DYKDDDDKGSGGSSDYKDDDDKS**PAATDN | 52 |
| G₃₂₂↑D₃₂₃ | GAADYK**DDDDKGSGGSSDYKDDDDKS**GAAGD | 33 |
| L₃₇₉↑A₃₈₀ | | 39 |
| L₄₃₆↑K₄₃₇ | LNAA**DYKDDDDKGSGGSSDYKDDDDKS**RPHLK | 67 |
| L₄₃₆↑K₄₃₇ | LMRP**DYKDDDDKGSGGSSDYKDDDDKS**PHLK | 77 |
| L₄₃₆↑K₄₃₇ | LNAA**DYKDDDDKGSGGSSDYKDDDDKS**RPHLK | 57 |
| F₄₄₄↑K₄₄₅ | FCGP**DYKDDDDKGSGGSSDYKDDDDKS**SRSFK | 70 |
| D₄₄₆↑I₄₄₇ | DAA**DYKDDDDKGSGGSSDYKDDDDKS**AKDI | 100 |
| I₄₄₇↑I₄₄₈ | IAA**DYKDDDDEGSGGSSDYKDDDDKS**PAADII | 88 |

## Claims

1. A fusion protein, capable of forming a Virus Like Particle, consisting of the incorporation at locations other than N- or C- terminus of one or more amino acid sequences relevant to human or veterinary health, other than an Infectious Bursal Disease Virus sequence, within a Birnaviridae virus VP2 protein, wherein preferably the VP2 protein is the VP2 protein of the Infectious Bursal Disease Virus or a VP2 that shares at least 30% amino acid sequence homology with the VP2 protein of the Infectious Bursal Disease Virus, and wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins.

2. A fusion protein according to claim 1, wherein the VP2 protein is a full length 512 amino acid pVP2, or 456, or 452 or 441 amino acid fragment thereof, or comprises at least 400 amino acids of VP2.

3. A fusion protein according to Claims 1 to 2 in which the amino acid sequences relevant to human or veterinary health are incorporated at one or more sites within the VP2 protein within the VP2 P loop regions BC (Q219-G224), DE (R249-G254), FG (T283-D287) and HI (S315-Q324), and/or outside said VP2 P loop regions, wherein the amino acid sequences relevant to human or veterinary health are the same or different for each incorporation.

4. A fusion protein according to Claims 1 to 3 wherein the incorporation of amino acid sequences relevant to human or veterinary health consists in insertions.

5. A fusion protein according to Claims 1 to 4 which is terminally fused either at its carboxy (C-) or amino (N-) terminal region, to a second amino acid sequence relevant to human or veterinary health which may be the same or different to the amino acid sequences relevant to human or veterinary health inserted in the P loop region or outside said P loop regions, wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins.

6. A Virus Like Particle constituted by assembly of any of the fusion proteins as defined in each of the Claims 1 to 5.

7. A Virus Like Particle constituted by assembly of the fusion proteins of Claim 6 and VP3 protein, wherein the VP3 protein is terminally fused, either at N- or C-terminus, to an amino acid sequence relevant to human or veterinary health which may be the same or different to those incorporated in VP2, wherein the amino acid sequences relevant to human or veterinary health are selected from the list consisting of: vaccine components, antigens and epitopes, targeting sequences, binding sequences, catalytic domains, pharmacology modulators, immunostimulators, toxins and antitoxins.

8. A Virus Like Particle according to Claims 6 to 7 which are obtained by the co-expression of pVP2 and VP3 from independent gene constructs or by the expression of constructs comprising the pVP2-VP4-VP3 polyprotein.

9. A nucleic acid encoding the fusion proteins defined in anyone of claims 1 to 5.

10. A gene construct comprising a nucleic acid according to Claim 9.

11. An expression system comprising a gene construct according to Claim 10 operatively bound to transcription, and optionally translation, control elements.

12. A host cell containing a nucleic acid according to Claim 9 or a gene construct according to Claim 10 or an expression system according to Claim 11.

13. The *in vitro* use of the gene expression system as defined in Claim 11 for producing Virus Like Particles.

14. The chimeric Virus Like Particles of Claims 6 to 8 for use in medicine, drug delivery, gene therapy, as targeted agents, imaging agents, in vaccination, as antitoxins, in diagnosis, or in imaging techniques applicable to human or veterinary health.

15. A screening method for the location of the preferred insertion sites within VP2 proteins, or fusion proteins thereof, efficiently assembling in to Virus Like Particles, which comprises:
a. Arranging, either individually or in pools, a set of any combination of DNA vectors library for Birnaviridae VP2 protein, or fusion proteins thereof, incorporating one or more vectors with preselected cloning sites or vectors with random insertion sites for the incorporation of DNA of interest leading to any of the VP2 fusion proteins according to Claims 1 to 5;
b. Contacting the set of DNA vectors of a) with a DNA of interest coding for an amino acid sequence relevant to human or veterinary health, for the incorporation of said DNA of interest, wherein the amino acid sequence relevant to human or veterinary health is selected from the list consisting of: vaccine components, antigens and epitopes, targeting sequences, binding sequences, catalytic domains, pharmacology modulators, immunostimulators, toxins and antitoxins;
c. Transfecting, transforming or infecting host cells with the DNA expression vectors resulting from b) for the expression of chimeric VP2 fusion proteins;
d. Evaluating the VLP formation efficiency of host cells from c) and selecting clones expressing chimeric Virus Like Particles with higher efficiencies;
e. Determining or confirming the preferred insertion location of the DNA of interest.

## Patentansprüche

1. Fusionsprotein, das ein Virus-ähnliches Partikel formen kann, bestehend aus dem Einbau an anderen Positionen als am N- oder C-Terminus einer oder mehrerer Aminosäuresequenzen, die für die Gesundheit von Mensch und Tier von Bedeutung sind, die eine andere Sequenz als die des infektiösen Bursitisvirus ist, innerhalb eines VP2-Proteins des Birnaviridae-Virus, wobei das VP2-Protein vorzugsweise das VP2-Protein des Virus der infektiösen Bursitis oder ein VP2, das mindestens 30 % Sequenzhomologie mit dem VP2-Protein des Virus der infektiösen Bursitis teilt, ist und wobei die Aminosäuresequenzen, die für die Gesundheit von Mensch und Tier von Bedeutung sind, von der Liste ausgewählt sind, bestehend aus: Impfstoffkomponenten, Antigenen und Epitopen, Zielsequenzen, Bindungssequenzen, katalytischen Domänen, pharmakologischen Modulatoren, Immunstimulatoren, Toxinen und Antitoxinen.

2. Fusionsprotein nach Anspruch 1, wobei das VP2-Protein ein 512 Aminosäure-pVP2 von voller Länge oder 456 oder 452 oder 441 Aminosäurefragment davon ist oder mindestens 400 Aminosäuren von VP2 umfasst.

3. Fusionsprotein nach den Ansprüchen 1 bis 2, in welchem die Aminosäuresequenzen, die für die Gesundheit von Mensch und Tier von Bedeutung sind, an einer oder mehreren Positionen innerhalb des VP2-Proteins in den VP2 P-Loop-Regionen BC (Q219-G224), DE (R249-G254), FG (T283-D287) und HI (S315-Q324) und/oder außerhalb der VP2 P-Loop-Regionen eingebaut sind, wobei die Aminosäuresequenzen, die für die Gesundheit von Mensch und Tier von Bedeutung sind, dieselben oder verschiedene für jeden Einbau sind.

4. Fusionsprotein nach den Ansprüchen 1 bis 3, wobei der Einbau von Aminosäuresequenzen, die für die Gesundheit von Mensch und Tier von Bedeutung sind, aus Insertionen besteht.

5. Fusionsprotein nach den Ansprüchen 1 bis 4, das endständig entweder an seiner Carboxy- (C-) oder Amino- (N-) terminalen Region an eine zweite Aminosäuresequenz fusioniert ist, die für die Gesundheit von Mensch und Tier von Bedeutung ist, welche dieselbe oder verschiedene wie die Aminosäuresequenzen sein kann, die für die Gesundheit von Mensch und Tier von Bedeutung sind, die in der P-Loop-Region oder außerhalb der P-Loop-Regionen eingefügt sind, wobei die Aminosäuresequenzen, die für die Gesundheit von Mensch und Tier von Bedeutung sind, aus der Liste ausgewählt sind, bestehend aus: Impfstoffkomponenten, Antigenen und Epitopen, Zielsequenzen, Bindungssequenzen, katalytischen Domänen, pharmakologischen Modulatoren, Immunstimulatoren, Toxinen und Antitoxinen.

6. Virus-ähnliches Partikel, das durch den Zusammenbau der Fusionsproteine nach Anspruch 1 bis 5 gebildet ist.

7. Virus-ähnliches Partikel, das durch den Zusammenbau der Fusionsproteine nach Anspruch 6 und VP3-Protein gebildet ist, wobei das VP3-Protein endständig entweder an dem N- oder C-Terminus an eine Aminosäuresequenz fusioniert ist, die für die Gesundheit von Mensch und Tier von Bedeutung ist, welche dieselbe oder verschiedene wie diese, die in VP2 eingebaut ist, sein kann, wobei die Aminosäuresequenzen, die für die Gesundheit von Mensch und Tier von Bedeutung sind, aus der Liste ausgwählt sind, bestehend aus: Impfstoffkomponenten, Antigenen und Epitopen, Zielsequenzen, Bindungssequenzen, katalytischen Domänen, pharmakologischen Modulatoren, Immunstimulatoren, Toxinen und Antitoxinen.

8. Virus-ähnliches Partikel nach Anspruch 6 bis 7, das durch die Coexpression von pVP2 und VP3 aus unabhängigen Genkonstrukten oder durch die Expression der Konstrukte, die das pVP2-VP4-VP3-Polyprotein umfassen, erhalten wird.

9. Nucleinsäure, welche die Fusionsproteine nach einem Ansprüche 1 bis 5 kodiert.

10. Genkonstrukt, das eine Nucleinsäure nach Anspruch 9 umfasst.

11. Expressionssystem, das ein Genkonstrukt nach Anspruch 10 umfasst, das operativ mit Transkriptions-, und gegebenenfalls Tranlations-, Steuerelementen verbunden ist.

12. Wirtszelle, die eine Nukleinsäure nach Anspruch 9 oder ein Genkonstrukt nach Anspruch 10 oder ein Expressionssystem nach Anspruch 11 enthält.

13. *In-vitro*-Verwendung des Genexpressionssystems nach Anspruch 11 zum Herstellen Virus-ähnlicher Partikel.

14. Chimäre Virus-ähnliches Partikel nach Anspruch 6 bis 8 zur Verwendung in Medizin, bei der Arzneimittelverabreichung, Gentherapie, als gezielte Wirkstoffe, bildgebende Mittel, bei der Impfung, als Antitoxine, bei der Diagnose oder bei bildgebenden Verfahren, die auf die Gesundheit von Mensch oder Tier anwendbar sind.

15. Screening-Verfahren zum Auffinden der bevorzugten Insertionsstellen innerhalb der VP2-Proteine oder Fusionsproteine davon, die wirksam zu Virus-ähnlichen Partikeln zusammengebaut werden, was Folgendes umfasst:
a. Anordnen, entweder einzeln oder in Pools, eines Satzes von einer Kombination einer DNA-Vektoren-Sammlung für das Birnaviridae-VP2-Protein oder von Fusionsproteinen davon, wobei ein oder mehrere Vektoren mit vorgewählten Klonierungsstellen für den Einbau von DNA von Interesse, welche zu einem der VP2-Fusionsproteine nach Anspruch 1 bis 5 führen, eingebaut werden;
b. In-Kontakt-Bringen des Satzes der DNA-Vektoren von a) mit einer DNA von Interesse, die für eine Aminosäuresequenz, die für die Gesundheit von Mensch oder Tier von Bedeutung ist, kodiert, für den Einbau der DNA von Interesse, wobei die Aminosäuresequenz, die für die Gesundheit von Mensch oder Tier von Bedeutung ist, ausgewählt ist aus der Liste, bestehend aus: Impfstoffkomponenten, Antigenen und Epitopen, Zielsequenzen, Bindungssequenzen, katalytischen Domänen, pharmakologischen Modulatoren, Immunstimulatoren, Toxinen und Antitoxinen;
c. Transfizieren, Umwandeln oder Infizieren von Wirtszellen mit den DNA-Expressionsvektoren aus b) für die Expression von chimären VP2-Fusionsproteinen;
d. Bewerten des VLP-Bildungswirkungsgrads der Wirtszellen von c) und Auswählen der Klone, die chimäre Virus-ähnliche Partikel mit höheren Wirkungsgraden exprimieren;
e. Bestimmen oder Bestätigen der bevorzugten Einbauposition der DNA von Interesse.

## Revendications

1. Protéine de fusion, capable de former une Pseudo-Particule Virale, consistant en l'incorporation à des localisations autres que les terminaisons N-ou C- de l'une ou plusieurs séquences d'acides aminés correspondant à la santé humaine ou animale, autres qu'une séquence virale de la maladie de la bursite infectieuse, au sein d'une protéine PV2 du virus *Birnaviridae,* dans laquelle, de préférence, la protéine PV2 est la protéine PV2 du virus de la maladie de la bursite infectieuse ou une PV2 qui partage au moins 30 % de l'homologie de la séquence d'acides aminés avec la protéine PV2 du virus de la maladie de la bursite infectieuse, et dans laquelle les séquences d'acides aminés correspondant à la santé humaine ou animale sont choisies dans la liste consistant en : des composants de vaccin, des antigènes et des épitopes, des séquences de ciblage, des séquences de liaison, des domaines catalytiques, des modulateurs pharmacologiques, des immunostimulateurs, des toxines et des antitoxines.

2. Protéine de fusion selon la revendication 1, dans laquelle la protéine PV2 est une pPV2 d'acide aminé 512 complète, ou fragment d'acide aminé 456, ou 452 ou 441 de celle-ci, ou comprend au moins 400 acides aminés de PV2.

3. Protéine de fusion selon les revendications 1 à 2 dans laquelle les séquences d'acides aminés correspondant à la santé humaine ou animale sont incorporées à un ou plusieurs sites au sein de la protéine PV2 au sein des régions de boucle P de PV2 BC (Q219-G224), DE (R249-G254), FG (T283-D287) et HI (S315-Q324), et/ou à l'extérieur desdites régions de boucle P PV2, dans lequel les séquences d'acides aminés correspondant à la santé humaine ou animale sont identiques ou différentes pour chaque incorporation.

4. Protéine de fusion selon les revendications 1 à 3 dans laquelle l'incorporation de séquences d'acides aminés correspondant à la santé humaine ou vétérinaire consiste en des insertions.

5. Protéine de fusion selon les revendications 1 à 4 qui est fusionnée à la terminaison à sa région carboxy (C-) ou amino (N-) terminale, à une deuxième séquence d'acides aminés correspondant à la santé humaine ou animale qui peut être identique ou différente des séquences d'acides aminés correspondant à la santé humaine ou animale insérées dans la région de boucle P ou à l'extérieur desdites régions de boucle P, dans laquelle les séquences d'acides aminés correspondant à la santé humaine ou animale sont choisies dans la liste consistant en : des composants de vaccin, des antigènes et des épitopes, des séquences de ciblage, des séquences de liaison, des domaines catalytiques, des modulateurs pharmacologiques, des immunostimulateurs, des toxines et des antitoxines.

6. Pseudo-Particule Virale constituée par l'assemblage de l'une quelconque des protéines de fusion tel qu'il est défini dans chacune des revendications 1 à 5.

7. Pseudo-Particule Virale constituée par l'assemblage des protéines de fusion selon la revendication 6 et la protéine PV3, dans laquelle la protéine PV3 est fusionnée à la terminaison, à la terminaison N- ou C-, à une séquence d'acides aminés correspondant à la santé humaine ou animale qui peut être identique ou différente de celles incorporées dans PV2, dans lequel les séquences d'acides aminés correspondant à la santé humaine ou animale sont choisies dans la liste consistant en : des composants de vaccin, des antigènes et des épitopes, des séquences de ciblage, des séquences de liaison, des domaines catalytiques, des modulateurs pharmacologiques, des immunostimulateurs, des toxines et des antitoxines.

8. Pseudo-Particules Virales selon les revendications 6 à 7 qui sont obtenues par la co-expression de pPV2 et PV3 à partir de constructions géniques indépendantes ou par l'expression de constructions comprenant la polyprotéine pPV2-PV4-PV3.

9. Acide nucléique encodant les protéines de fusion définies dans l'une quelconque des revendications 1 à 5.

10. Construction génique comprenant un acide nucléique selon la revendication 9.

11. Système d'expression comprenant une construction génique selon la revendication 10 reliée de façon fonctionnelle aux éléments de contrôle de transcription, et optionnellement de traduction.

12. Cellule hôte contenant un acide nucléique selon la revendication 9 ou une construction génique selon la revendication 10 ou système d'expression selon la revendication 11.

13. Utilisation *in vitro* du système d'expression génique tel qu'il est défini dans la revendication 11 pour produire des Pseudo-Particules Virales.

14. Pseudo-Particules virales chimériques selon les revendications 6 à 8 pour leur utilisation dans la médecine, l'administration de médicaments, la thérapie génique, comme les agents ciblés, les agents d'imagerie, dans la vaccination, tels que les antitoxines, dans le diagnostic, ou dans les techniques d'imagerie applicables à la santé humaine ou animale.

15. Procédé de dépistage pour la localisation de sites d'insertion préférés au sein des protéines PV2, ou des protéines de fusion de celles-ci, les assemblant efficacement dans les Pseudo-Particules Virales, qui comprend :
a. La disposition, soit individuellement soit dans des pools, d'un ensemble de n'importe quelle combinaison de bibliothèque de vecteurs ADN pour la protéine PV2 de *Birnaviridae,* ou les protéines de fusion de celle-ci, incorporant un ou plusieurs vecteurs avec des sites ou vecteurs de clonage pré-choisis avec des sites d'insertion aléatoires pour l'incorporation d'ADN d'intérêt conduisant à l'une quelconque des protéines de fusion PV2 selon les revendications 1 à 5 ;
b. L'entrée en contact de l'ensemble de vecteurs ADN de a) avec un ADN d'intérêt codant une séquence d'acides aminés correspondant à la santé humaine ou animale, pour l'incorporation dudit ADN d'intérêt, dans laquelle la séquence d'acides aminés correspondant à la santé humaine ou animale est choisie dans la liste consistant en : des composants de vaccin, des antigènes et des épitopes, des séquences de ciblage, des séquences de liaison, des domaines catalytiques, des modulateurs pharmacologiques, des immunostimulateurs, des toxines et des antitoxines ;
c. La transfectation, la transformation ou l'infection des cellules hôtes avec les vecteurs d'expression ADN découlant de b) pour l'expression de protéines de fusion PV2 chimérique ;
d. L'évaluation de l'efficacité de la formation de PPV de cellules hôtes provenant de c) et le choix de clones exprimant les Pseudo-Particules Virales chimériques ayant des efficacités élevées ;
e. La détermination ou la confirmation de la localisation d'insertion préférée de l'ADN d'intérêt.


### SEQUENCE LISTING

<110> CHIMERA PHARMA, S.L.U
<120> Chimeric fusion proteins and virus like particles from Birnavir VP2
<130> Application Reference
<160> 26
<170> PatentIn version 3.3
<210> 1
   <211> 2
   <212> PRT
   <213> Artificial
<220>
   <223> TS peptide
<400> 1
   Thr Ser
   1
<210> 2
   <211> 23
   <212> PRT
   <213> Artificial
<220>
   <223> Flag peptide
<400> 2
   Asp Tyr Lys Asp Asp Asp Asp Lys Gly Ser Gly Gly Ser Ser Asp Tyr
   1 5 10 15
   Lys Asp Asp Asp Asp Lys Ser
   20
<210> 3
   <211> 11
   <212> PRT
   <213> Artificial
<220>
   <223> cMyc peptide
<400> 3
   Glu Gln Lys Leu Ile Ser Glu Glu Asp Leu Ser
   1 5 10
<210> 4
   <211> 15
   <212> PRT
   <213> Artificial
<220>
   <223> V5 peptide
<400> 4
   Gly Lys Pro Ile Pro Asn Pro Leu Leu Gly Leu Asp Ser Thr Ser
   1 5 10 15
<210> 5
   <211> 12
   <212> PRT
   <213> Artificial
<220>
   <223> VSV-G peptide
<400> 5
   Tyr Thr Asp Ile Glu Met Asn Arg Leu Gly Lys Ser
   1 5 10
<210> 6
   <211> 34
   <212> DNA
   <213> Artificial
<220>
   <223> Oligonucleotide VP2-452EcoRI-fw
<400> 6
   gcccgaattc atgacaaacc tgtcagatca aacc 34
<210> 7
   <211> 36
   <212> DNA
   <213> Artificial
<220>
   <223> Oligonucleotide VP2-452NotI-rev
<400> 7
   gcccgcggcc gcttacctta tggcccggat tatgtc 36
<210> 8
   <211> 39
   <212> DNA
   <213> Artificial
<220>
   <223> Oligonucleotide VP2 456-rev
<400> 8
   gcccgcggcc gcttacacag ctatcctcct tatggcccg 39
<210> 9
   <211> 39
   <212> DNA
   <213> Artificial
<220>
   <223> Oligonucleotide VP2 441-rev
<400> 9
   gcccgcggcc gcttatgctc ctgcaatctt caggggaga 39
<210> 10
   <211> 32
   <212> DNA
   <213> Artificial
<220>
   <223> Oligonucleotide BV VP3 5' HindIII
<400> 10
   ctgaaagctt tcactcaagg tcctcatcag ag 32
<210> 11
   <211> 65
   <212> DNA
   <213> Artificial
<220>
   <223> Oligonucleotide BV VP3-Flag 3'HindIII
<400> 11
   ctgaaagctt tcatttatca tcatcatctt tataatcacc tgatgactca aggtcctcat 60
   cagag 65
<210> 12
   <211> 62
   <212> DNA
   <213> Artificial
<220>
   <223> Oligonucleotide BV VP3-c-Myc 3' HindIII
<400> 12
   ctgaaagctt tcacaaatct tcttcggaaa tcaatttttg ttcctcaagg tcctcatcag 60
   ag 62
<210> 13
   <211> 60
   <212> DNA
   <213> Artificial
<220>
   <223> Oligonucleotide Y His-VP3 5'
<400> 13
   atcgcggccg catgcatcat catcatcatc acagcagcgg cgctgcatca gagttcaaag 60
<210> 14
   <211> 32
   <212> DNA
   <213> Artificial
<220>
   <223> Oligonucleotide Y VP3 3'
<400> 14
   ctgagagctc tcactcaagg tcctcatcag ag 32
<210> 15
   <211> 65
   <212> DNA
   <213> Artificial
<220>
   <223> Oligonucleotide Y VP3-Flag 3'
<400> 15
   ctgagagctc tcatttatca tcatcatctt tataatcacc tgatgactca aggtcctcat 60
   cagag 65
<210> 16
   <211> 62
   <212> DNA
   <213> Artificial
<220>
   <223> Oligonucleotide Y VP3-cMyc 3'
<400> 16
   ctgagagctc tcacaaatct tcttcggaaa tcaatttttg ttcctcaagg tcctcatcag 60
   ag 62
<210> 17
   <211> 59
   <212> DNA
   <213> Artificial
<220>
   <223> Oligonucleotide BV His-VP3 5'
<400> 17
   atcgcccggg atgcatcatc atcatcatca cagcagcggc gctgcatcag agttcaaag 59
<210> 18
   <211> 30
   <212> DNA
   <213> Artificial
<220>
   <223> Oligonucleotide BV VP3 3'
<400> 18
   ctgaggtacc tcactcaagg tcctcatcag 30
<210> 19
   <211> 65
   <212> DNA
   <213> Artificial
<220>
   <223> Oligonucleotide BV VP3-Flag 3'
<400> 19
   ctgaggtacc tcatttatca tcatcatctt tataatcacc tgatgactca aggtcctcat 60
   cagag 65
<210> 20
   <211> 62
   <212> DNA
   <213> Artificial
<220>
   <223> Oligonucleotide BV VP3-cMyc 3'
<400> 20
   ctgaggtacc tcacaaatct tcttcggaaa tcaatttttg ttcctcaagg tcctcatcag 60
   ag 62
<210> 21
   <211> 1536
   <212> DNA
   <213> Artificial
<220>
   <223> synthetic pVP2 gene
<400> 21
<210> 22
   <211> 512
   <212> PRT
   <213> Artificial
<220>
   <223> synthetic pVP2 protein
<400> 22
   Met Thr Asn Leu Ser Asp Gln Thr Gln Gln Ile Val Pro Phe Ile Arg
   1 5 10 15
   Ser Leu Leu Met Pro Thr Thr Gly Pro Ala Ser Ile Pro Asp Asp Thr
   20 25 30
   Leu Glu Lys His Thr Leu Arg Ser Glu Thr Ser Thr Tyr Asn Leu Thr
   35 40 45
   Val Gly Asp Thr Gly Ser Gly Leu Ile Val Phe Phe Pro Gly Phe Pro
   50 55 60
   Gly Ser Ile Val Gly Ala His Tyr Thr Leu Gln Gly Asn Gly Asn Tyr
   65 70 75 80
   Lys Phe Asp Gln Met Leu Leu Thr Ala Gln Asn Leu Pro Ala Ser Tyr
   85 90 95
   Asn Tyr Cys Arg Leu Val Ser Arg Ser Leu Thr Val Arg Ser Ser Thr
   100 105 110
   Leu Pro Gly Gly Val Tyr Ala Leu Asn Gly Thr Ile Asn Ala Val Thr
   115 120 125
   Phe Gln Gly Ser Leu Ser Glu Leu Thr Asp Val Ser Tyr Asn Gly Leu
   130 135 140
   Met Ser Ala Thr Ala Asn Ile Asn Asp Lys Ile Gly Asn Val Leu Val
   145 150 155 160
   Gly Glu Gly Val Thr Val Leu Ser Leu Pro Thr Ser Tyr Asp Leu Gly
   165 170 175
   Tyr Val Arg Leu Gly Asp Pro Ile Pro Ala Ile Gly Leu Asp Pro Lys
   180 185 190
   Met Val Ala Thr Cys Asp Ser Ser Asp Arg Pro Arg Val Tyr Thr Ile
   195 200 205
   Thr Ala Ala Asp Asp Tyr Gln Phe Ser Ser Gln Tyr Gln Pro Gly Gly
   210 215 220
   Val Thr Ile Thr Leu Phe Ser Ala Asn Ile Asp Ala Ile Thr Ser Leu
   225 230 235 240
   Ser Val Gly Gly Glu Leu Val Phe Arg Thr Ser Val His Gly Leu Val
   245 250 255
   Leu Gly Ala Thr Ile Tyr Leu Ile Gly Phe Asp Gly Thr Thr Val Ile
   260 265 270
   Thr Arg Ala Val Ala Ala Asn Asn Gly Leu Thr Thr Gly Thr Asp Asn
   275 280 285
   Leu Met Pro Phe Asn Leu Val Ile Pro Thr Asn Glu Ile Thr Gln Pro
   290 295 300
   Ile Thr Ser Ile Lys Leu Glu Ile Val Thr Ser Lys Ser Gly Gly Gln
   305 310 315 320
   Ala Gly Asp Gln Met Ser Trp Ser Ala Arg Gly Ser Leu Ala Val Thr
   325 330 335
   Ile His Gly Gly Asn Tyr Pro Gly Ala Leu Arg Pro Val Thr Leu Val
   340 345 350
   Ala Tyr Glu Arg Val Ala Thr Gly Ser Val Val Thr Val Ala Gly Val
   355 360 365
   Ser Asn Phe Glu Leu Ile Pro Asn Pro Glu Leu Ala Lys Asn Leu Val
   370 375 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Ile Ile
   435 440 445
   Arg Ala Ile Arg Arg Ile Ala Val Pro Val Val Ser Thr Leu Phe Pro
   450 455 460
   Pro Ala Ala Pro Leu Ala His Ala Ile Gly Glu Gly Val Asp Tyr Leu
   465 470 475 480
   Leu Gly Asp Glu Ala Gln Ala Ala Ser Gly Thr Ala Arg Ala Ala Ser
   485 490 495
   Gly Lys Ala Arg Ala Ala Ser Gly Arg Ile Arg Gln Leu Thr Leu Ala
   500 505 510
<210> 23
   <211> 840
   <212> DNA
   <213> Artificial
<220>
   <223> synthetic VP4 gene
<400> 23
   aggcagctga ctctcgccgc cgacaagggg tacgaggtag tcgcgaatct attccaggtg 60
   ccccagaatc ccgtagtcga cgggattctt gcttcacctg gggtactccg cggtgcacac 120
   aacctcgact gcgtgttaag agagggtgcc acgctattcc ctgtggttat tacgacagtg 180
   gaagacgcca tgacacccaa agcattgaac agcaaaatgt ttgctgtcat tgaaggcgtg 240
   cgagaagacc tccaacctcc atctcaaaga ggatccttca tacgaactct ctctggacac 300
   agagtctatg gatatgctcc agatggggta cttccactgg agactgggag agactacacc 360
   gttgtcccaa tagatgatgt ctgggacgac agcattatgc tgtccaaaga tcccatacct 420
   cctattgtgg gaaacagtgg aaatctagcc atagcttaca tggatgtgtt tcgacccaaa 480
   gtcccaatcc atgtggctat gacgggagcc ctcaatgctt gtggcgagat tgagaaagta 540
   agctttagaa gcaccaagct cgctactgcg caccgacttg gccttaggtt ggctggtccc 600
   ggagcattcg atgtaaacac cgggcccaac tgggcaacgt tcatcaaacg tttccctcac 660
   aatccacgcg actgggacag gctcccctac ctcaacctac cataccttcc acccaatgca 720
   ggacgccagt accaccttgc catggctgca tcagagttca aagagacccc cgaactcgag 780
   agtgccgtca gagcaatgga agcagcagcc aacgtggacc cactattcca atctgcactc 840
<210> 24
   <211> 280
   <212> PRT
   <213> Artificial
<220>
   <223> synthetic VP4 protein
<400> 24
   Arg Gln Leu Thr Leu Ala Ala Asp Lys Gly Tyr Glu Val Val Ala Asn
   1 5 10 15
   Leu Phe Gln Val Pro Gln Asn Pro Val Val Asp Gly Ile Leu Ala Ser
   20 25 30
   Pro Gly Val Leu Arg Gly Ala His Asn Leu Asp Cys Val Leu Arg Glu
   35 40 45
   Gly Ala Thr Leu Phe Pro Val Val Ile Thr Thr Val Glu Asp Ala Met
   50 55 60
   Thr Pro Lys Ala Leu Asn Ser Lys Met Phe Ala Val Ile Glu Gly Val
   65 70 75 80
   Arg Glu Asp Leu Gln Pro Pro Ser Gln Arg Gly Ser Phe Ile Arg Thr
   85 90 95
   Leu Ser Gly His Arg Val Tyr Gly Tyr Ala Pro Asp Gly Val Leu Pro
   100 105 110
   Leu Glu Thr Gly Arg Asp Tyr Thr Val Val Pro Ile Asp Asp Val Trp
   115 120 125
   Asp Asp Ser Ile Met Leu Ser Lys Asp Pro Ile Pro Pro Ile Val Gly
   130 135 140
   Asn Ser Gly Asn Leu Ala Ile Ala Tyr Met Asp Val Phe Arg Pro Lys
   145 150 155 160
   Val Pro Ile His Val Ala Met Thr Gly Ala Leu Asn Ala Cys Gly Glu
   165 170 175
   Ile Glu Lys Val Ser Phe Arg Ser Thr Lys Leu Ala Thr Ala His Arg
   180 185 190
   Leu Gly Leu Arg Leu Ala Gly Pro Gly Ala Phe Asp Val Asn Thr Gly
   195 200 205
   Pro Asn Trp Ala Thr Phe Ile Lys Arg Phe Pro His Asn Pro Arg Asp
   210 215 220
   Trp Asp Arg Leu Pro Tyr Leu Asn Leu Pro Tyr Leu Pro Pro Asn Ala
   225 230 235 240
   Gly Arg Gln Tyr His Leu Ala Met Ala Ala Ser Glu Phe Lys Glu Thr
   245 250 255
   Pro Glu Leu Glu Ser Ala Val Arg Ala Met Glu Ala Ala Ala Asn Val
   260 265 270
   Asp Pro Leu Phe Gln Ser Ala Leu
   275 280
<210> 25
   <211> 780
   <212> DNA
   <213> Artificial
<220>
   <223> synthetic VP3 gene
<400> 25
   atggctgcat cagagttcaa agagaccccc gaactcgaga gtgccgtcag agcaatggaa 60
   gcagcagcca acgtggaccc actattccaa tctgcactca gtgtgttcat gtggctggaa 120
   gagaatggga ttgtgactga catggccaac ttcgcactca gcgacccgaa cgcccatcgg 180
   atgcgaaatt ttcttgcaaa cgcaccacaa gcaggcagca agtcgcaaag ggccaagtac 240
   gggacagcag gctacggagt ggaggctcgg ggccccacac cagaggaagc acagagggaa 300
   aaagacacac ggatctcaaa gaagatggag accatgggca tctactttgc aacaccagaa 360
   tgggtagcac tcaatgggca ccgagggcca agcccaggcc aggtaaagta ctggcagaac 420
   aaacgagaaa taccggaccc aaacgaggac tatctagact acgtgcatgc agagaagagc 480
   cggttggcat cagaagaaca aatcctaagg gcagctacgt cgatctacgg ggctccagga 540
   caggcagagc caccccaagc tttcatagac gaagttgcca aagtctatga aatcaaccat 600
   ggacgtggcc caaaccaaga acagatgaaa gatctgctct tgactgcgat ggagatgaag 660
   catcgcaatc ccaggcgggc tctaccaaag cccaagccaa aacccaatgc tccaacacag 720
   agaccccctg gtcggctggg ccgctggatc aggaccgtct ctgatgagga ccttgagtga 780
<210> 26
   <211> 259
   <212> PRT
   <213> Artificial
<220>
   <223> synthetic VP3 protein
<400> 26
   Met Ala Ala Ser Glu Phe Lys Glu Thr Pro Glu Leu Glu Ser Ala Val
   1 5 10 15
   Arg Ala Met Glu Ala Ala Ala Asn Val Asp Pro Leu Phe Gln Ser Ala
   20 25 30
   Leu Ser Val Phe Met Trp Leu Glu Glu Asn Gly Ile Val Thr Asp Met
   35 40 45
   Ala Asn Phe Ala Leu Ser Asp Pro Asn Ala His Arg Met Arg Asn Phe
   50 55 60
   Leu Ala Asn Ala Pro Gln Ala Gly Ser Lys Ser Gln Arg Ala Lys Tyr
   65 70 75 80
   Gly Thr Ala Gly Tyr Gly Val Glu Ala Arg Gly Pro Thr Pro Glu Glu
   85 90 95
   Ala Gln Arg Glu Lys Asp Thr Arg Ile Ser Lys Lys Met Glu Thr Met
   100 105 110
   Gly Ile Tyr Phe Ala Thr Pro Glu Trp Val Ala Leu Asn Gly His Arg
   115 120 125
   Gly Pro Ser Pro Gly Gln Val Lys Tyr Trp Gln Asn Lys Arg Glu Ile
   130 135 140
   Pro Asp Pro Asn Glu Asp Tyr Leu Asp Tyr Val His Ala Glu Lys Ser
   145 150 155 160
   Arg Leu Ala Ser Glu Glu Gln Ile Leu Arg Ala Ala Thr Ser Ile Tyr
   165 170 175
   Gly Ala Pro Gly Gln Ala Glu Pro Pro Gln Ala Phe Ile Asp Glu Val
   180 185 190
   Ala Lys Val Tyr Glu Ile Asn His Gly Arg Gly Pro Asn Gln Glu Gln
   195 200 205
   Met Lys Asp Leu Leu Leu Thr Ala Met Glu Met Lys His Arg Asn Pro
   210 215 220
   Arg Arg Ala Leu Pro Lys Pro Lys Pro Lys Pro Asn Ala Pro Thr Gln
   225 230 235 240
   Arg Pro Pro Gly Arg Leu Gly Arg Trp Ile Arg Thr Val Ser Asp Glu
   245 250 255
   Asp Leu Glu